# EUROPEAN PATENT APPLICATION

(11) **EP 4 691 382 A1**
(43) Date of publication of application: **11.02.2026**
(21) Application number: 24723925.4
(22) Date of filing: 02.04.2024
(51) Int. Cl.: A61B 17/16, A61B 17/17

(54) **RUPTURING TOOL WITH A MOVABLE PIVOT BODY AND RUPTURING SYSTEM COMPRISING SAID RUPTURING TOOL**

(30) Priority: 03.04.2023 EP 23382320
(71) Applicant: Abanza Tecnomed, S.L., 31192 Mutilva, Navarra (ES)
(72) Inventor: ABASCAL RUBIO, José Manuel, 31007 Pamplona, Navarra (ES); ABASCAL AZANZA, Juan, 31006 Pamplona, Navarra (ES); MENAUT BELTRAN, Ander, 20018 San Sebastián, Guipúzcoa (ES); MAYA PABOLAZA, Alejandro, 31002 Pamplona, Navarra (ES)
(74) Representative: ABG Intellectual Property Law, S.L.
(86) International application number: PCT/ES2024/070205
(87) International publication number: WO 2024/209126

(57) **Abstract**

The present invention relates to a rupturing tool for surgical interventions and to a rupturing system comprising the rupturing tool and a rupturing movement generator device. The rupturing tool and system are intended for the field of traumatology, particularly for the creation of bone tunnels suitable for the anatomical reconstruction of tendons, for example, for the creation of bone tunnels in interventions for reinserting the supraspinatus muscle tendon of the rotator cuff in the shoulder joint.

## Description

### OBJECT OF THE INVENTION

The present invention relates to a rupturing tool for surgical interventions and to a rupturing system comprising the rupturing tool and a rupturing movement generator device. The rupturing tool and the rupturing system are intended for the field of traumatology, particularly for the creation of bone tunnels suitable for the anatomical reconstruction of tendons; for example, for the creation of bone tunnels in interventions for reinserting the supraspinatus muscle tendon of the rotator cuff in the shoulder joint.

### BACKGROUND OF THE INVENTION

One of the most common injuries in the field of traumatology is joint tendon ruptures.

Among the most common tendon ruptures are tendon rupture in the rotator cuff of the shoulder, mainly the supraspinatus muscle tendon. This injury represents the main cause of shoulder instability and is present in 20.7% of the general population, with a prevalence that increases with age (*"*Prevalence and risk factors of a rotator cuff tear in the general population", Yamamoto A et al / J Shoulder Elbow Surg. 2010 Jan; 19(1):116-20*).*

Anchorings which are inserted into the head of the humerus are often used during supraspinatus tendon reconstruction surgery. These anchorings carry suture threads or suture bands, which are passed through the damaged end of the tendon, in order to pull on it, reposition it, and fix it on the original area of insertion.

Document US10206672B2 discloses an arthroscopic bone channel forming and suturing system comprising: a punch configured to form a first generally straight channel in a bone; a drill configured to form a second generally straight channel in said bone, said second generally straight channel not intersecting said first generally straight channel; a curved needle configured to be inserted into said first generally straight channel; a needle driving assembly configured to manipulate said curved needle to form a curved junction between said first generally straight channel and said second generally straight channel; and a suture assembly configured to insert a suture to a suture pick-up location via said second generally straight channel in said bone. Said curved needle being configured to pull said suture from said suture pick up location and through said junction and said first generally straight channel.

Although document US10206672B2 mentions an arthroscopic curved needle that can be introduced in an arcuate manner into a bone tunnel previously created by means of a punch, it is not suitable for use as a rupturing element. The needle is only an insertion element but not a rupturing element. Also, the curved needle disclosed in US10206672B2 has only a forward (linear) movement, never a rotary movement, as it is inserted in a straight tunnel.

In many cases, a problem which prevents a successful reconstruction is that the anchorings carrying the sutures or bands only provide surface compression at the end of the tendon, on the original area of insertion of the tendon, an area which is furthermore reduced by the anchoring itself, such that the healing process is hindered. If the tendon does not heal, the surgery is inadequate because it is not only intended for repositioning the tendon in the bone, but also for promoting the tendon-bone healing process for a proper reconstruction of said damaged tendon (Castagna et al. 2018, Arthroscopic Transosseous Rotator Cuff Repair*).*

The solution to this problem requires creating an angled transosseous tunnel by means of minimally invasive surgery and then widening the intraarticular outlet opening to provide a bone housing along the original anatomical area of insertion, intended for the insertion of the damaged end of the tendon and/or of the augmentation tissue used for repair.

Therefore, there is a need to provide rupturing tools and rupturing systems which, by means of minimally invasive surgeries, allow widening, in a precise, reliable, and reproducible manner, the intraarticular outlet opening of an angled bone tunnel along the original anatomical area of insertion of the tendon being repaired; successfully providing a bone housing suitable for the anatomical reinsertion and regeneration of the end of a damaged tendon or ligament.

### DESCRIPTION OF THE INVENTION

The present invention proposes a solution to the preceding problem by means of a rupturing tool for surgical interventions according to claim 1 and rupturing systems for surgical interventions according to claims 12 and 13. The dependent claims define preferred embodiments of the invention.

In a first inventive aspect, the invention provides *a rupturing tool for minimally invasive surgical interventions, comprising:*
- *a tubular main body which in turn comprises: a longitudinal proximal portion, an intermediate portion, and* a *distal tip, wherein the intermediate portion connects the longitudinal proximal portion and the distal tip;*
- *a rupturing assembly which comprises:*
   ∘ a *first rupturing element which is configured to receive and perform a rupturing movement about a first axis of rupture; and*
   ∘ a *movement transfer element, housed inside the main body, which is configured to receive and perform a rupturing movement and to transfer said rupturing movement to the first rupturing element; wherein the movement transfer element comprises a proximal portion and a distal portion; and wherein the movement transfer element is attached to the first rupturing element by the distal portion;*
- *a longitudinal pivot body, oriented substantially along a pivot axis; and*
- *sliding coupling means adapted to allow the movement of the longitudinal pivot body along the pivot axis; wherein said sliding coupling means comprise at least a first component and a second component; the first component being adapted to be housed at least partially in the second component and to be able to move inside the second component, or vice versa, the second component being adapted to move while housing the first component;*

*wherein in said rupturing tool:*
   - *the pivot axis and the axis of rupture define a first angle (α) comprised substantially in the range between 20° and 170°; and*
   - *the axis of rupture and the pivot axis define a sagittal plane of the rupturing tool which contains both axes in any position of the rupturing tool; and*
*wherein the rupturing tool is adapted to swivel from a first position to at least a second position by means of the rotation of the sagittal plane with respect to the pivot axis; such that after swiveling from the first position to the second position:*
   - *the sagittal plane in the at least second position defines a second angle (β) with respect to the sagittal plane in the first position, and*
   - *the first rupturing element in the at least second position defines said second angle (β) with respect to the position of said first rupturing element in the first position.*

Among other uses, the rupturing tool of the invention allows the use thereof in minimally invasive surgical operations in which there is a need to create bone tunnels suitable for the anatomical reconstruction of damaged tendons in the bone insertion thereof. In the scope of the present description, minimally invasive surgical operations refer to arthroscopic interventions. Nevertheless, the use of the rupturing tool of the invention is not limited to interventions of this type.

**In** particular, the tool allows creating bone tunnels with elongated intraarticular outlet openings, similar to the original anatomical areas of insertion, capable of housing the tendon being repaired at a sufficient bone depth such that it ensures tendon regeneration. Restoration which favors osseointegration is thereby achieved.

Throughout this document, tendons shall be understood to mean bands of connective tissue configured to attach muscles to bones. In the scope of the present description, the concept of "tendon" shall not be understood to mean only "natural" tendons according to their usual definition (as bands of connective tissue), but also artificial patches or synthetic tissues that come into play in minimally invasive surgeries of this type when the band of connective tissue present has a small or insufficient size.

Throughout this document, the proximal end of an element of the rupturing tool (or of the system) shall be understood to mean the end closest to the subject who is using said tool. In contrast, the distal end of an element of the rupturing tool (or of the system) shall be understood to mean the end farthest away from the subject who is using said tool. Preferably, the subject or user of the rupturing tool is a doctor, a veterinarian, or some type of medical or veterinary professional.

Additionally, in the scope of the present invention, the term "substantially" in relation to an angle will refer to the fact that the value of said angle is "exactly" said value or within a tolerance margin of ±1°.

In the scope of this invention, sagittal plane shall be understood to mean a plane comprising the pivot axis and the axis of rupture. Said sagittal plane splits the tool, from the perspective of a user using said tool, into two halves ("left" and "right" according to the figures that will be shown below).

Likewise, in the context of this invention, the longitudinal pivot body shall be understood to mean a substantially elongated element arranged along a pivot axis, adapted to pivot at least partially to both sides of a pivot axis; and it is also adapted to move along said pivot axis.

In the invention, the rupturing tool is adapted to swivel from a first position to at least a second position by means of the rotation of the sagittal plane with respect to the pivot axis. For this purpose, in the invention the rupturing element receives from the movement transfer element a rupturing movement that allows the breaking of the bone tissue by means of a "windshield wiper" sweep wherein the pivot axis works as the axis of rotation.

Concerning this invention, the sliding coupling means comprise at least a first component and a second component, said first and second components being complementary to one another. In said embodiments, one of the components (e.g., the first component) is housed (at least partially) in at least the other component (e.g., the second component), and at the same time, said sliding coupling means allow one of the components to move with respect to the other. The complementary sliding coupling means are adapted to allow the movement of the longitudinal pivot body along the pivot axis.

In preferred embodiments of the rupturing tool of the invention, said sliding coupling means are tongued and grooved means, with at least one male component and one female component. These tongued and grooved means constitute a particular case of sliding coupling means with complementary components, wherein the first component is a male component and the second component is a female component. In these embodiments, the male component is movably housed in at least one female component (i.e., the male component is at least partially housed inside the female component and can move inside said female component), or vice versa (i.e., the female component can move while it at least partially houses the male component).

First, the rupturing tool comprises a main body which in turn comprises a longitudinal proximal portion. "Longitudinal" shall be understood to mean that the body is made or placed in its lengthwise direction. Preferably, the main body is tubular; that is, a tube-shaped hollow body generally open at both ends.

Advantageously, in a particular preferred embodiment of the invention, intended for repairing the supraspinatus tendon, the curved and/or angled intermediate portion of the rupturing tool comprises a geometry and dimensions similar to the contour of the head of the humerus.

The axis of rupture and the pivot axis of the rupturing tool are comprised in a first plane of the rupturing tool. The tubular main body in turn comprises: a longitudinal proximal portion, an intermediate portion, and a distal tip. The intermediate portion can be curved and/or angled.

Throughout the document, a "rupturing element" shall be understood to mean any type of element capable of rupturing, milling, cutting, reaming, polishing, compacting, or perforating a biological tissue; particularly bone tissue.

The first rupturing element protrudes at least partially through the opening of the distal tip towards the outside of the tool.

In a particular example of the rupturing tool, the first rupturing element can be one of the following: a mill, a rupturing and/or compacting mill, a bit, a blade, a scraper, a rasp, a vibrating rupturing and/or compacting element, or an oscillating rupturing and/or compacting element, or a reciprocating rupturing and/or compacting element.

In another particular example, the first rupturing element is a piezoelectric and/or ultrasound rupturing element.

Advantageously, in a preferred embodiment, the first rupturing element is a compacting mill which causes osseodensification which is important for early loading, allowing the rehabilitation time required to return to the pre-injury activity level to be shortened.

In other more particular examples, the first rupturing element:
- has a frustoconical geometry, with a proximal diameter greater than the distal diameter;
- has a cylindrical geometry;
- is a mixed element, wherein the proximal segment is a compacting segment and the distal segment is a rupturing segment;
- has a circular, elliptical, trilobular, polygonal distal cross-section, or a distal cross-section corresponding to any other geometric figure.

This first rupturing element is oriented substantially along an axis of rupture; for example, an axis of rotation about which the element performs rotation, oscillating rotation, or an axis of vibration about which the element performs vibrating movement. Said first rupturing element is configured to receive and perform a rupturing movement, with this being understood to mean a movement that can be performed by the rupturing element such that said element can rupture a human tissue, preferably bone. Examples of rupturing movement include rotational and/or oscillating rotational and/or vibrating movement performed about the first axis of rupture.

Moreover, the tool comprises a rupturing assembly. Said assembly in turn comprises the first rupturing element and a movement transfer element attached to one another. The movement transfer element is housed (completely or at least partially) inside the main body.

The rupturing tool of the present invention can be considered a contra-angle tool, since the axis of rupture and pivot axis are not coaxial, but rather form a certain angle substantially other than 0°, specifically between 20° and 170° (both ends included).

As will be described in detail below, other particular embodiments of the rupturing tool have multiple rupturing elements. In such embodiments, any of the different rupturing elements can be an element from the preceding lists, both with respect to the geometry and with respect to the type of actuation (piezoelectric, ultrasound, etc.).

In a particular embodiment, the first angle α between the axis of rupture and the pivot axis is an angle comprised substantially between 20° and 170°.

More particularly, in a preferred application of the invention for the anatomical widening of the intraarticular outlet opening of a bone tunnel in the humerus for repairing the supraspinatus tendon, the first angle is 90°, which presents the advantage of allowing the guaranteed avoidance of the axillary nerve, but the drawback of the bone plug possibly being insufficient.

In another alternative particular embodiment, said first angle (α) is substantially 135°. Advantageously, this value of the first angle provides a larger bone plug, and also without the risk of damaging the axillary nerve.

The second angle (β) of the rupturing tool depends mainly on three factors: the first angle (α), the length of the rupturing element and of the particular anatomy of the subject subjected to the minimally invasive intervention. In preferred embodiments, the second angle is comprised in the range of 70°-120°.

Advantageously, the rupturing tool allows a user of the tool to angle the rupturing element at will by a desired value of the second angle.

In a preferred embodiment of the rupturing tool, *the movement transfer element further comprises at least a first attachment and*/*or coupling means configured to attach and*/*or couple the movement transfer element to the first rupturing element; and wherein said movement transfer element is attached to a second attachment and*/*or coupling means configured to attach and*/*or couple the movement transfer element to a rupturing movement generator device.*

In anticipation of the probable existence of many types of different rupturing movement generator devices, the option of the rupturing tool further comprises one or more attachment and/or coupling means configured to couple the main body and the movement transfer element to one or more of said rupturing movement generator devices is contemplated. In certain embodiments of the invention, the rupturing tool is adapted to cooperate with a drill which acts as a rupturing movement generator device.

In any of the embodiments of the rupturing tool of the invention, both the first attachment and/or coupling means and the second attachment and/or coupling means can be a hexagonal female, a male hexagon, a cube, etc., such as any conventional key coupling.

Advantageously, the tool is even more versatile, as it can be coupled by means of the corresponding attachment and/or coupling means to any rupturing movement generator device that may be commercially available.

In a preferred embodiment, this movement transfer element comprises two parts or portions: a proximal part, which can be both rigid and flexible; and a flexible or articulated distal part firmly attached to a proximal end of the first rupturing element.

In a preferred embodiment, the flexibility of the movement transfer element is due to a braided tubular configuration of the element. In another example, the flexibility of the movement transfer element is due to a tubular configuration comprising cuts and/or openings in the element. In another example, the flexibility of the movement transfer element is due to said element comprising a superelastic nitinol braided core and an outer polymer layer.

Advantageously, the rupturing tool comprises sliding coupling means for the longitudinal pivot body for the sliding thereof along the longitudinal proximal portion of the tubular main body according to the pivot axis.

In a preferred embodiment of the rupturing tool, *the sliding coupling means for the longitudinal pivot body further comprise blocking means for the longitudinal pivot body configured to act on said longitudinal pivot body in one or more longitudinal positions along the pivot axis, and wherein said blocking means are configured to enable at least two positions of the longitudinal pivot body:*
- *a blocked position in which the longitudinal position of the longitudinal pivot body is fixed with respect to the longitudinal proximal portion, and*
- *an unblocked position in which the longitudinal pivot body can move linearly along the longitudinal proximal portion.*

In any of the embodiments of the rupturing tool comprising blocking means for the longitudinal pivot body, said blocking means in turn comprise:
- a brake element linked to the female component which is adapted to press on the male component, or
- a brake element linked to the male component which presses on the female component.

In the scope of the present invention, "brake element" shall be understood to mean any mechanical element which is associated/linked/attached to one of the components of the sliding coupling means (e.g., the female component) and allows pressing/acting on the other component of said sliding coupling means (e.g., the male component). In preferred embodiments, said brake element is a trigger, a tab, an appendage, etc.

In another preferred embodiment of the rupturing tool, *the longitudinal pivot body further comprises:*
- *a longitudinal pivot element arranged substantially along the pivot axis;*
- *a first handle attached to the longitudinal pivot element; and*

*a first through conduit attached to the longitudinal pivot element; wherein said first through conduit is adapted to house the longitudinal proximal portion and to allow the linear movement* of *said longitudinal proximal portion along the pivot axis;*
*wherein the first component of the sliding coupling means is a male component, and the second component of the sliding coupling means is a female component; and*
*wherein the male component of the sliding coupling means comprises the longitudinal proximal portion of the tubular main body, and the female component of said sliding coupling means comprises the first through conduit.*

Advantageously, the first through conduit is a tubular element which allows housing the longitudinal proximal portion slidingly along the pivot axis.

The first handle allows the user to pivot the main body about the pivot axis at will between a plurality of positions for each of which the angulation of the sagittal plane of the rupturing tool changes (i.e., the second angle (β) is modified).

Throughout this description, the second handle, third handle, and fourth handle, respectively, shall be referred to as the incorporated handle in certain preferred embodiments of the invention. Nevertheless, functionally, all the handles perform the same function as the first handle described above.

Likewise, the use of this embodiment of the rupturing tool requires a prior milling of the angled bone tunnel by means of a rupturing guide, as will be seen below.

In another preferred embodiment of the rupturing tool, *the longitudinal pivot body further comprises:*
- *a longitudinal channel which contains the pivot axis, wherein said longitudinal channel comprises a face surrounding the longitudinal proximal portion of the tubular main body; wherein the longitudinal channel is adapted to slide along the longitudinal proximal portion;*
- *a second handle attached to the longitudinal channel; and*
- *a second through conduit attached to the longitudinal pivot element, wherein said second through conduit is adapted to house the longitudinal proximal portion and to allow the linear movement of said longitudinal proximal portion along the pivot axis;*

*wherein the first component of the sliding coupling means is a male component, and the second component of the sliding coupling means is a female component; and*
*wherein the male component of the sliding coupling means comprises the longitudinal proximal portion of the tubular main body, and the female component* of *said sliding coupling means comprises the second through conduit.*

Advantageously, the second through conduit (which is preferably tubular) slidingly houses the longitudinal proximal portion. The blocking means for the longitudinal pivot body are adapted to allow a bidirectional blocking of the longitudinal movement.

The use of this embodiment of the rupturing tool requires a prior milling of the angled bone tunnel by means of a rupturing guide, as will be seen below.

In another preferred embodiment of the rupturing tool, *the blocking means for the longitudinal pivot body comprise:*
- *a trigger adapted to fit into a longitudinal groove of the longitudinal proximal portion; and*
- *a push spring attached to the trigger, said push spring being configured to exert an actuation force on the trigger which keeps the longitudinal pivot body in the blocked position pressing the tubular main body, when the trigger is not pressed;*
*and wherein the trigger is configured so that when a user presses it, it opposes the actuation force of the spring, allowing the unblocking of the pivot body on the longitudinal groove, and so that said pivot body can move longitudinally along the pivot axis.*

In this embodiment, blocking is generated by the trigger impacting against the tubular main body. In particular, the tubular main body comprises a longitudinal groove or rail on a lower surface of said tubular main body. An upper tab of the trigger is housed in the longitudinal groove. Said longitudinal groove comprises an upper face and side faces. When the trigger is not subject to actuation (for example, by a user who presses said trigger), the longitudinal groove and the trigger are in contact, preventing the movement between both. When the trigger is actuated, the tab no longer contacts the upper face of the longitudinal groove, such that the tubular main body can move in a sliding manner; and at the same time the tab always contacts the side faces of the longitudinal groove (otherwise the rotation of the rupturing tool would not be allowed).

In another preferred embodiment of the rupturing tool, *the pivot body further comprises:*
- *a third through conduit which in turn comprises at least one longitudinal groove adapted to fit at least one longitudinal projection of the longitudinal proximal portion, the third through conduit being adapted to house the longitudinal proximal portion and to allow the linear movement of said longitudinal proximal portion along the pivot axis;*
- *a fourth through conduit; and*
- *a second rupturing element, partially housed in the fourth through conduit and adapted to project from said fourth through conduit, wherein said second rupturing element comprises a second attachment and*/*or coupling means which are adapted to attach or couple a proximal end of the second rupturing element to the rupturing movement generator device; and*
*wherein:*
- *the tubular main body further comprises a third handle;*
- *the second rupturing element is configured to rotate freely inside the fourth through conduit about a fixed longitudinal position of said fourth through conduit; and*
- *the second rupturing element is configured to move integrally with the pivot body along the pivot axis;*
- *the first component of the sliding coupling means is a male component, and the second component of the sliding coupling means is a female component; and*
- *the male component of the sliding coupling means comprises the longitudinal proximal portion and the longitudinal projection; and the female component of said sliding coupling means comprises the third through conduit and the longitudinal groove.*

The second rupturing element is housed at least partially in the fourth through conduit.

In another preferred embodiment of the rupturing tool, *the longitudinal pivot body further comprises:*
- *a first striking edge, a prolongation of the longitudinal pivot body; wherein said first striking edge is configured to receive a striking force in the direction of the pivot axis;*
- *a fifth through conduit which in turn comprises at least one longitudinal groove adapted to fit at least one longitudinal projection of the longitudinal proximal portion; and said fifth through conduit also comprises a perpendicular groove adapted to house a rotation projection;*
- *a sixth through conduit adapted to house and guide a bit;*

*wherein the first component of the sliding coupling means is a male component, and the second component of the sliding coupling means is a female component; and*
*wherein the male component of the sliding coupling means comprises the longitudinal proximal portion and the longitudinal projection; and the female component of said sliding coupling means comprises the fifth through conduit and the longitudinal groove;*
*wherein the tubular main body further comprises a fourth handle attached to the longitudinal proximal portion; and*
*wherein the longitudinal pivot body is configured to perform a linear movement, moving along the longitudinal proximal portion from at least one rotation-blocking position to at least one rotation position, or vice versa, wherein:*
   - *if the longitudinal pivot body is located in the at least one rotation-blocking position, the longitudinal groove houses at least one portion of a longitudinal projection, and the perpendicular groove does not house any portion of the longitudinal projection; and*
   - *if the longitudinal pivot body is located in the at least one rotation position, the longitudinal groove does not house any portion of the longitudinal projection, and the perpendicular groove houses the rotation projection.*

Therefore, in the rotation-blocking position, the longitudinal pivot body cannot rotate about the pivot axis. Rotation-blocking position shall be understood to mean a position in which at least a portion of the longitudinal projection is housed in the longitudinal groove.

In contrast, in the rotation position, the longitudinal pivot body can rotate at least partially about the pivot axis. In particular, in the rotation position, the longitudinal groove has completely overcome the longitudinal projection, and the perpendicular groove houses the rotation projection. In certain even more preferred embodiments, said rotation projection of the longitudinal proximal portion is hemispherical.

For example, the first striking edge (as well as the other striking edges featured in the embodiments of the rupturing tool) can be a piece adhered to the longitudinal pivot body or integrated in said longitudinal pivot body as a single piece.

Advantageously, the force applied to the first striking edge ensures that the distal portion of the pivot body is first driven into the bone cortex. This allows the pivot body to remain in a stable position, which increases the carving precision of the first segment of the bone tunnel in the target anatomical position, and further improves the actual usability of the tool. Preferably, the proximal end of the first striking edge is substantially flat.

Second, the force applied to the first striking edge ensures that the distal portion of the pivot body snugly penetrates the first segment of the previously carved bone tunnel with the pivot body transitioning to a rotation position suitable for the anatomical widening of the second segment of the bone tunnel.

In a preferred embodiment, linear movement can also exist in the rotation position.

In other embodiments, and by way of a safety mechanism, in the rotation position there is an angular stop which limits the second angle of rotation of the pivot body about the pivot axis.

In another preferred embodiment of the rupturing tool, *the first axis of rupture and the pivot axis are arranged in the rupturing tool:*
- *defining a fixed first angle (α); or*
- *defining a variable first angle (α) according to a predetermined pattern.*

For example, the predetermined pattern can be determined by a criterion established by the user of the tool or by an automated control system.

In a particular embodiment, the rupturing tool comprises variable coupling means between the intermediate portion and the longitudinal proximal portion of the tubular main body which allow the user to vary the first angle (α) between the axis of rupture and the pivot axis at will.

In preferred embodiments, the striking edge(s) of the rupturing tool comprise(s) a substantially flat portion.

In another even more preferred embodiment of the rupturing tool, said tool *further comprises a second attachment and*/*or coupling means configured to attach and*/*or couple, in a fixed position, the longitudinal proximal portion of the tubular main body to the rupturing movement generator device; such that in such situation, the rupturing tool is integrally attached to the rupturing movement generator device.*

Advantageously, the rupturing tool and the rupture generator device only fit in a certain position (the fixed position). Evidently, the pivot body is not fixed with respect to the rupturing movement generator device since it must be able to slide freely with respect to the tubular main body in order to be able to properly perform its function.

In another alternative embodiment of the rupturing tool, *at least the distal portion of the movement transfer element is a flexible longitudinal element housed in the intermediate portion.* Said flexible longitudinal element must be able to be housed in the curved and/or angled intermediate portion. By way of illustration, the flexible longitudinal element can be a cable, a braided cable, a flexible metal guide, a chain, etc.

In an embodiment of the rupturing tool, the first rupturing element is straight.

In an embodiment of the rupturing tool, the rupturing movement is rotary, oscillating rotary and/or vibrating about the first rupturing axis (which is a straight rupturing axis). Advantageously, in this embodiment the movement transfer element transfers rotary or vibratory motion to the rupturing element, but not linear motion.

Within the scope of the present invention, two types of movements will be defined for the rupturing movement: rotary and oscillating.

Rotary motion is a rotational motion around a fixed axis. A particular case of rotary motion is defined as reciprocating rotary motion.

Oscillating motion is a back-and-forth motion around a stable equilibrium position, so that it moves, preferably periodically, between two extreme positions. Specific examples of oscillating motion include vibratory, ultrasonic or piezoelectric motion.

As a combination of the above two types of motion (i.e. rotary and oscillating), the rotary oscillating motion of a rupturing element can be defined as the rupturing element is configured to rotate in one direction a certain angle, and then rotate in the opposite direction, repeating the change of direction continuously.

When the oscillating motion is linear, it is also called linear reciprocating motion.

On the other hand, when the mechanical waves that are transmitted are of higher frequency than those of sound, it is called ultrasonic vibratory motion. For example, in dental surgery, the piezoelectric scalpel is used as a surgical tool that, through ultrasound, is able to cut bone tissue without damaging the soft tissues attached to it.

In other preferred embodiments of the rupturing tool where there is more than one rupturing element and more than one rupturing axis (see for example embodiment C of the detailed description, comprising two rupturing elements), several of the rupturing elements are configured to receive rotary, oscillating rotary and/or vibratory rupturing motion.

In a second inventive aspect, the invention provides a first *rupturing system for minimally invasive surgical interventions, comprising:*
- *a rupturing movement generator device configured to generate and perform a rupturing movement; and*
- *at least one rupturing tool according to any of the preceding claims adapted to cooperate with the rupturing movement generator device; said rupturing tool being able to be coupled to the rupturing movement generator device at least by means of the movement transfer element;*
*and wherein the rupturing movement generator device is further configured to transfer the rupturing movement to the movement transfer element.*

Advantageously, the movement transfer element works as a mechanical coupling to transfer the rupturing movement generated by the rupturing movement generator device to the rupturing tool. This facilitates the reutilization of the rupturing movement generator device with different types of rupturing tools.

In a third inventive aspect, the invention provides a second *rupturing system for minimally invasive surgical interventions, comprising:*
- *a rupturing movement generator device configured to generate and perform a rupturing movement; and*
- *a rupturing tool according to any of the preceding claims integrally attached to the rupturing movement generator device by means of the movement transfer element and by means of the longitudinal proximal portion;*
*wherein the rupturing movement generator device is further configured to transfer the rupturing movement to the movement transfer element.*

According to the second and third inventive aspects, the invention not only contemplates the rupturing tool, but also a rupturing system, wherein the rupturing tool and a rupturing movement generator device are arranged together and cooperate with one another.

All the advantages derived from the rupturing tool of the first inventive aspect can be extrapolated to any of the rupturing systems comprising said rupturing tool.

In one embodiment, the rupturing tool of the system according to the second or third inventive aspect is disposable. In another alternative or additional embodiment, at least one part of the rupturing movement generator device is disposable.

In another preferred embodiment of the rupturing systems according to the second or third inventive aspect, *the rupturing movement generated and performed with the rupturing movement generator device is a rotational movement.*

Throughout the document, rotary or rotational movement shall be understood to mean movement in which the rupturing element performs a (constant or non-constant) 360-degree rotation. In a particular embodiment, the rupturing movement is an oscillating rotary movement, this oscillating rotary movement being understood as the rupturing element being configured to rotate a certain angle in one direction, and then rotating in the opposite direction, repeating the change in direction continuously. In other embodiments, the rupturing movement is a vibratory movement. Vibrating or vibratory movement shall be understood to mean movement in which the rupturing element performs a preferably periodic oscillating movement about a stable equilibrium position, such that it moves between two end positions.

In a preferred embodiment, particularly suitable for use when the rupturing movement generated and performed with the rupturing movement generator device is a rotational movement, the rupturing tool further comprises a transmission and conversion means for transmitting rotary movement and converting it into oscillating rotary movement, wherein said transmission and conversion means in turn comprise:
- a proximal end and a distal end arranged longitudinally on a drive shaft;
- an eccentric distal projection which is eccentric with respect to the drive shaft, and
- a rotary body which in turn comprises a longitudinal groove and a coupling portion configured to couple said rotary body to the first rupturing element; wherein said rotary body is adapted to rotate about the drive shaft;
wherein the eccentric distal projection is adapted to fit into the longitudinal groove of the rotary body;
said transmission and conversion means being configured to:
- receive the rotational movement from a rupturing movement generator device, wherein said rupturing movement is a rotational movement;
- convert the rotational movement into a reciprocating rotational movement;
- transmit the reciprocating rotary movement to the movement transfer element such that said movement transfer element transfers the reciprocating rotary movement to the first rupturing element, causing it to move from a first position to the at least a second position;
- cease the reciprocating rotary movement when the rotational movement ceases, causing the rupturing element to move from the at least a second position to the first position.

In a preferred embodiment of the rupturing system according to the second or third inventive aspect, said rupturing system *further comprises a rupturing guide adapted to carve a bone tunnel, wherein the rupturing guide comprises:*
- *a first tubular guide which in turn comprises:*
   ∘ a *first longitudinal conduit oriented according to a first longitudinal guide axis, wherein the first longitudinal conduit comprises a first proximal end and a first distal end; and*
   ∘ a *second striking edge, a prolongation of the first longitudinal conduit at the first proximal end; wherein the second striking edge is configured to receive a striking force in the direction of the first longitudinal guide axis;*
- *a second tubular guide which in turn comprises:*
   ∘ a *second longitudinal conduit oriented according to a second longitudinal guide axis, wherein the second longitudinal conduit comprises a second proximal end and a second distal end; and*
   ∘ a *third striking edge, a prolongation of the second longitudinal conduit at the second proximal end; wherein the third striking edge is configured to receive a striking force in the direction of the second longitudinal guide axis; and*
- *a guide arch which comprises:*
   ∘ *a proximal arch portion which in turn comprises a first sliding coupling means adapted to couple and uncouple, in a fixed manner, the proximal arch portion with respect to the first tubular guide in a plurality of different positions along the first longitudinal guide axis; and*
   ∘ *a distal arch portion which in turn comprises a second sliding coupling means adapted to couple and uncouple, in a fixed manner, the distal arch portion with respect to the second tubular guide in a plurality of different positions along the second longitudinal guide axis;*
*wherein in the rupturing guide:*
- *the first longitudinal conduit and the second longitudinal conduit are adapted to accommodate a bit, said bit being configured to move along said longitudinal conduits;*
- *the first longitudinal guide axis and the second longitudinal guide axis form an angle substantially equal to the first angle (α) of the rupturing tool with respect to one another.*

The first coupling means for coupling, in a fixed manner, the proximal arch portion to the first tubular guide in a plurality of different positions along the first longitudinal guide axis, as well as the second coupling means for coupling, in a fixed manner, the distal arch portion to the second tubular guide in a plurality of different positions along the first longitudinal guide axis advantageously allow the rupturing guide to be very versatile and to be able to adapt to a specific (medical or veterinary) application, to a particular surgery, and/or to the particular anatomy of a patient which involves the creation of an angled bone tunnel. Another additional advantage is that the guide arch and the tubular guides can be uncoupled with ease, which facilitates the removal of said guide arch once the angled bone tunnel is created.

In a particular embodiment, any of the rupturing systems of the invention is a one-piece system.

In a particular example of use, the rupturing tool of the present invention is suitable for creating and/or carving of all types of tunnels in surgical interventions for the reconstruction of any connective tissue and/or cartilage tissue and/or bone tissue, in medicine or veterinary science.

Lastly, an advantageous technical effect of any of the embodiments of the present invention is that the anatomical widenings created with the rupturing tool greatly favor tissue healing, which lead to an effective operation and a long-lasting result.

All the features and/or the steps of the methods described in this specification (including the claims, description, and drawings) can be combined in any combination, except for combinations of such mutually exclusive features.

### DESCRIPTION OF THE DRAWINGS

These and other features and advantages of the invention will be shown more clearly based on the following detailed description of a preferred embodiment, provided solely by way of illustrative and non-limiting example in reference to the attached figures.
Figures 1A-1H show multiple views of an embodiment of the rupturing tool according to the present invention ("Embodiment A").
Figures 2A-2D show multiple views of an embodiment of the rupturing tool according to the present invention ("Embodiment B").
Figures 3A-3D show multiple views of an embodiment of the rupturing tool according to the present invention ("Embodiment C").
Figures 4A-4J show multiple views of an embodiment of the rupturing tool according to the present invention ("Embodiment D").
Figures 5A-5B show two views of the rupturing guide of a system according to the present invention.
Figure 6 show the different geometries of the rupturing elements of the rupturing tool of the present invention.
Figures 7A-7C show multiple views of a first rupturing system according to the present invention, in which the rupturing tool and the rupturing movement generator device are attached by the movement transfer element.
Figures 8A-8C show multiple views of a second rupturing system according to the present invention, in which the rupturing tool and the rupturing movement generator device are attached by the movement transfer element and by the tubular main body.
Figures 9A-9C schematically illustrate the steps of a method for carving an angled bone tunnel.

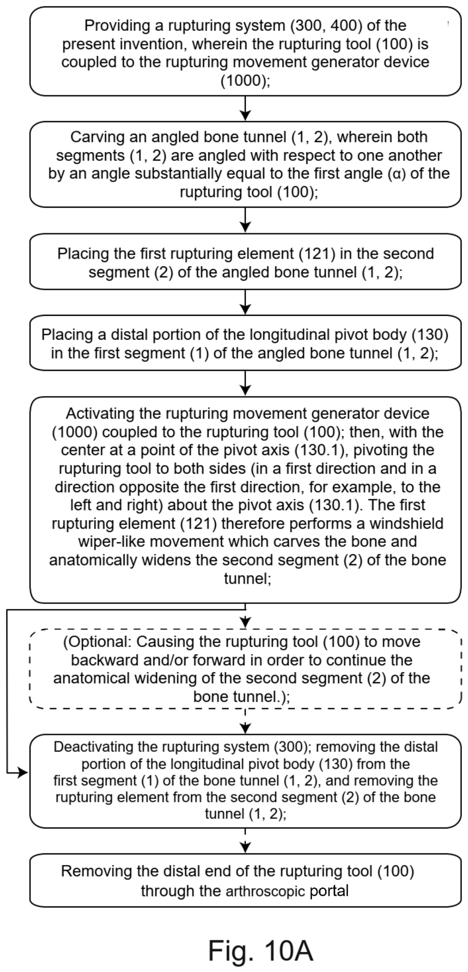

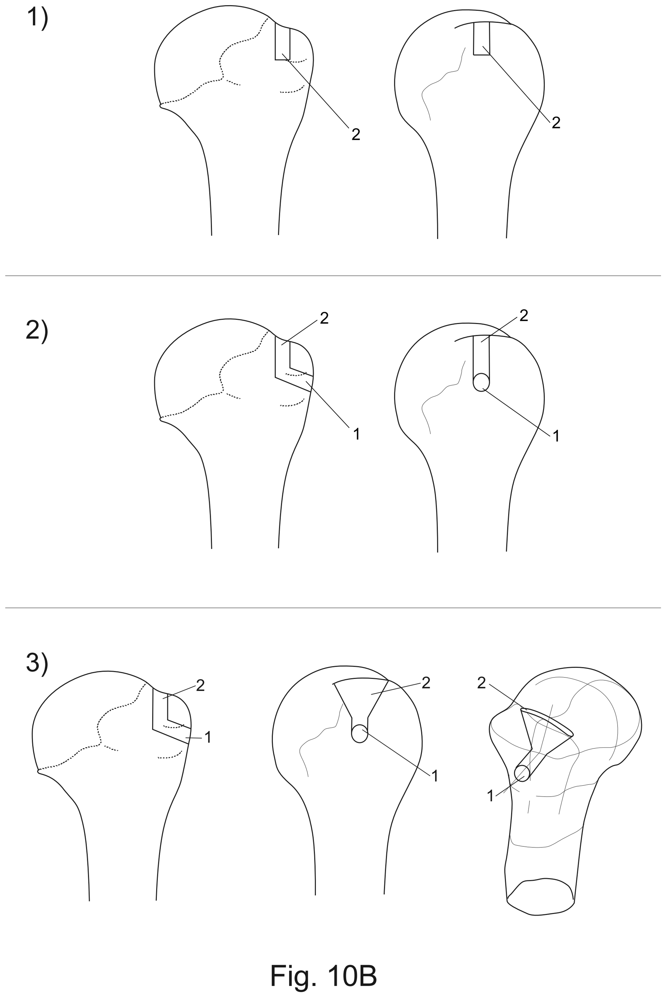

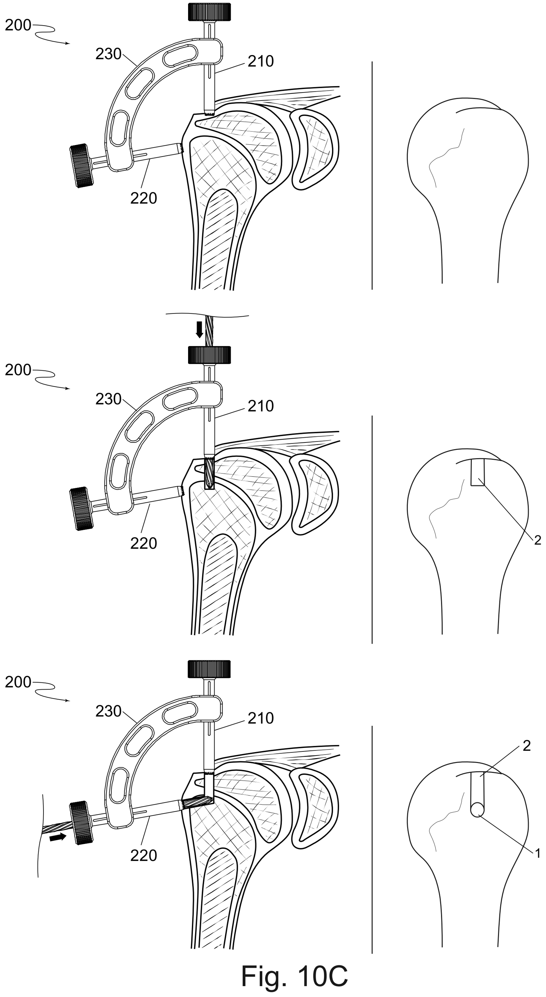

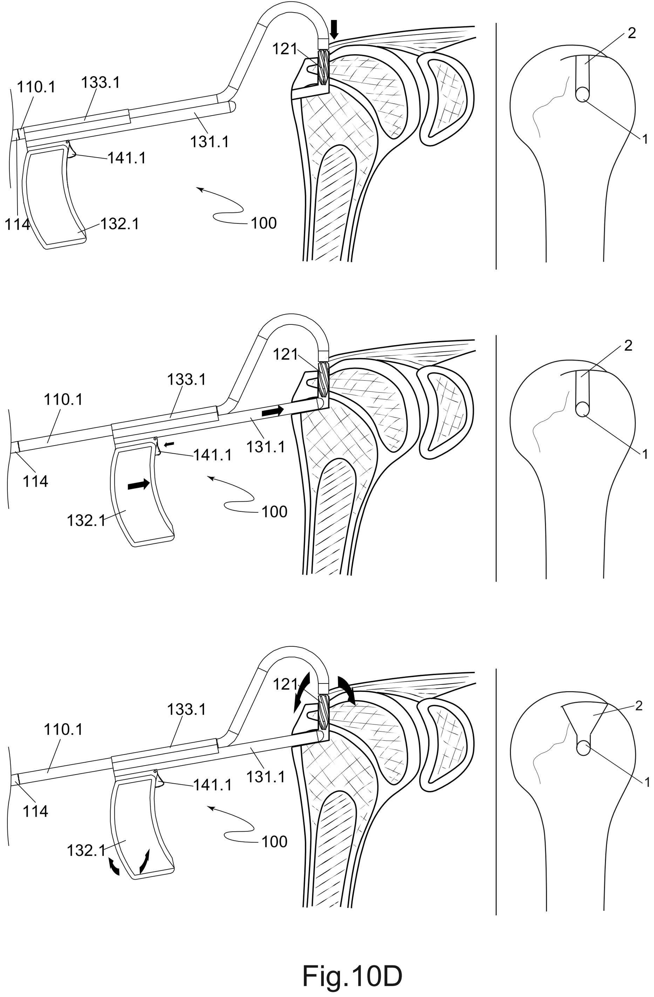

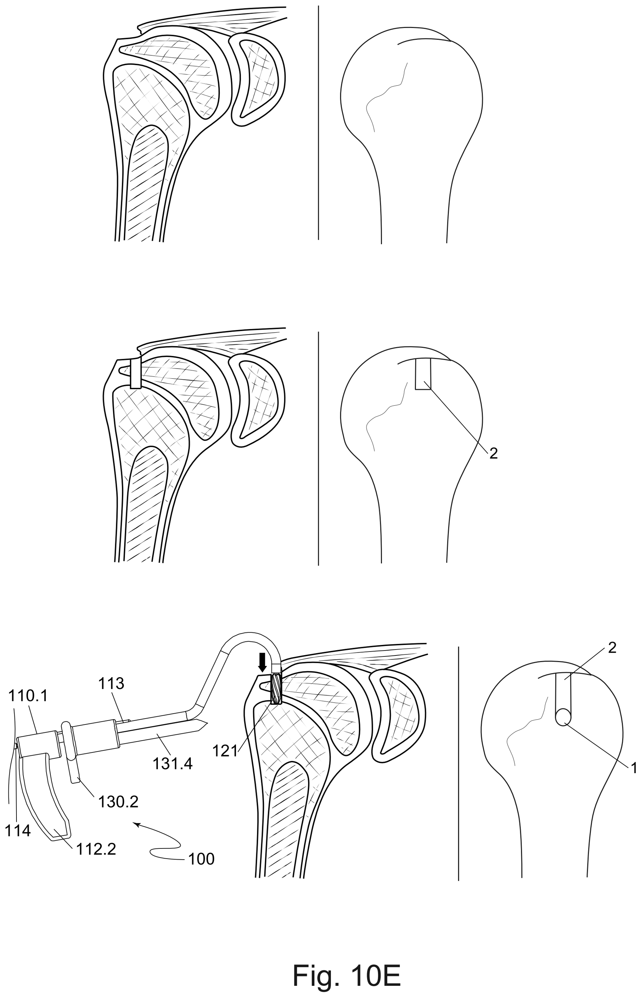

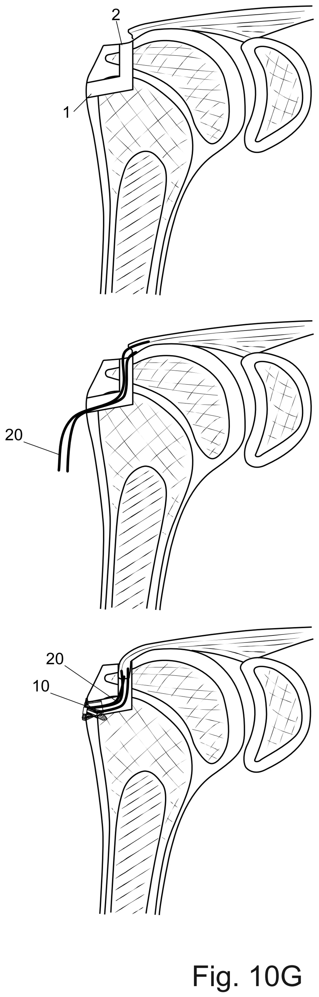

Figures 10A-10G correspond to the steps of a method for carving an angled bone tunnel with an intraarticular outlet opening widened using the rupturing tool of the invention. Figure 10A is a flow chart of said method. Figure 10B shows a musculoskeletal depiction of the bone tunnel carved with one of the rupturing systems of the invention, and the insertion of a graft for repairing a supraspinatus muscle tendon of the shoulder joint. Figures 10C, 10D, and 10G show a first example of use of the rupturing tool according to Embodiment C, for repairing the supraspinatus tendon of the shoulder joint. Figures 10E-10G illustrate a second example of use of the rupturing tool according to Embodiment D, for repairing the supraspinatus tendon of the shoulder joint.
Figures 11A-11B schematically illustrate how to repair a partial rupture (Figure 11A) and a total rupture (Figure 11B) of the supraspinatus muscle tendon.

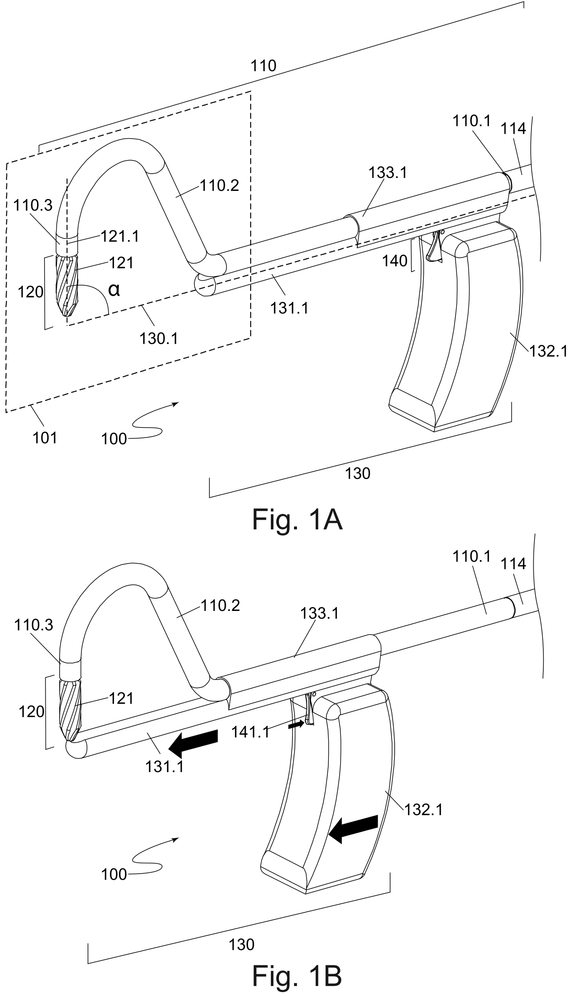

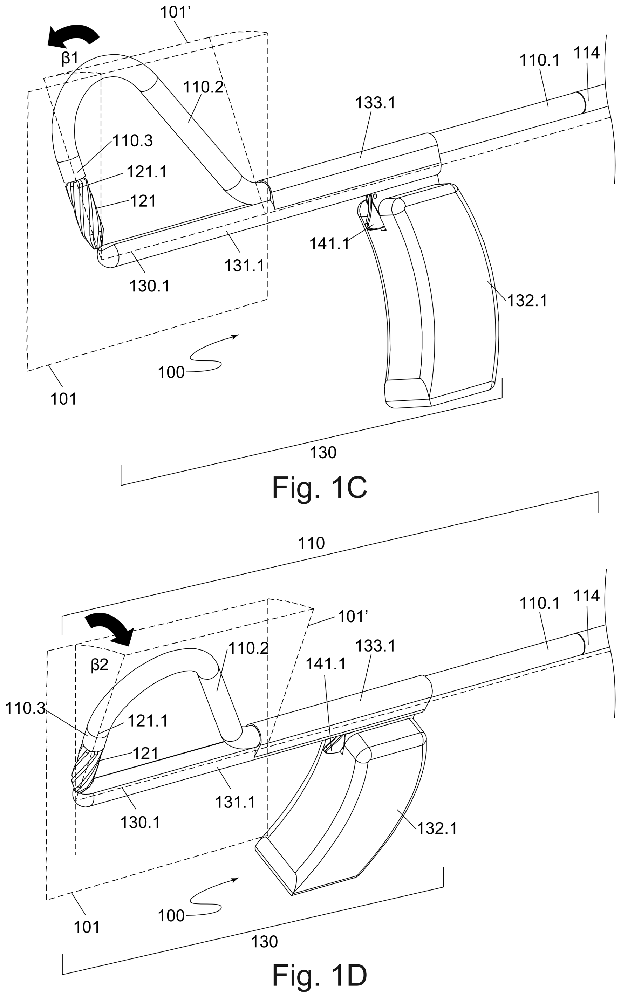

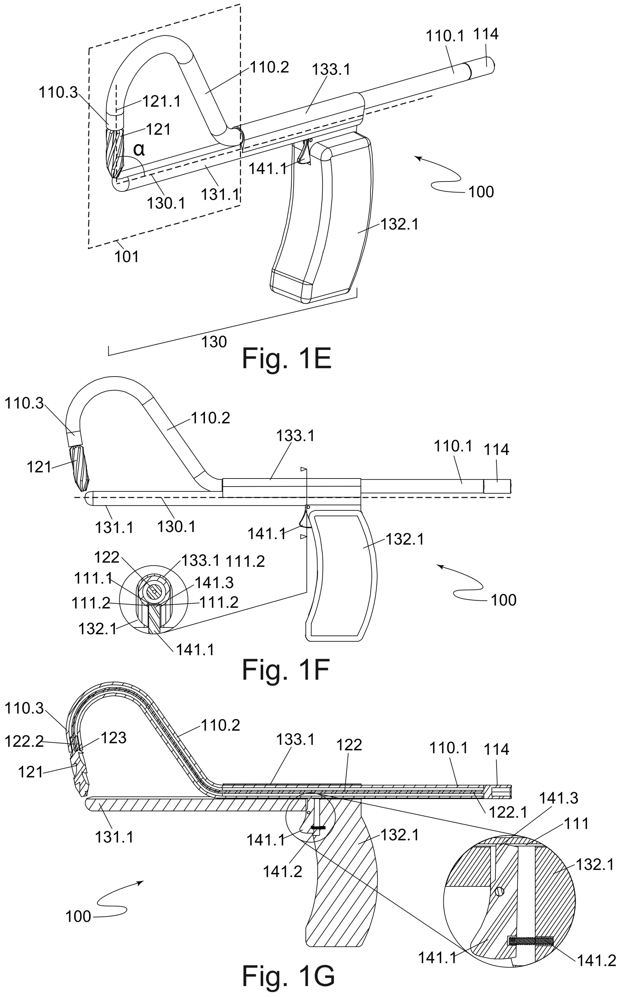

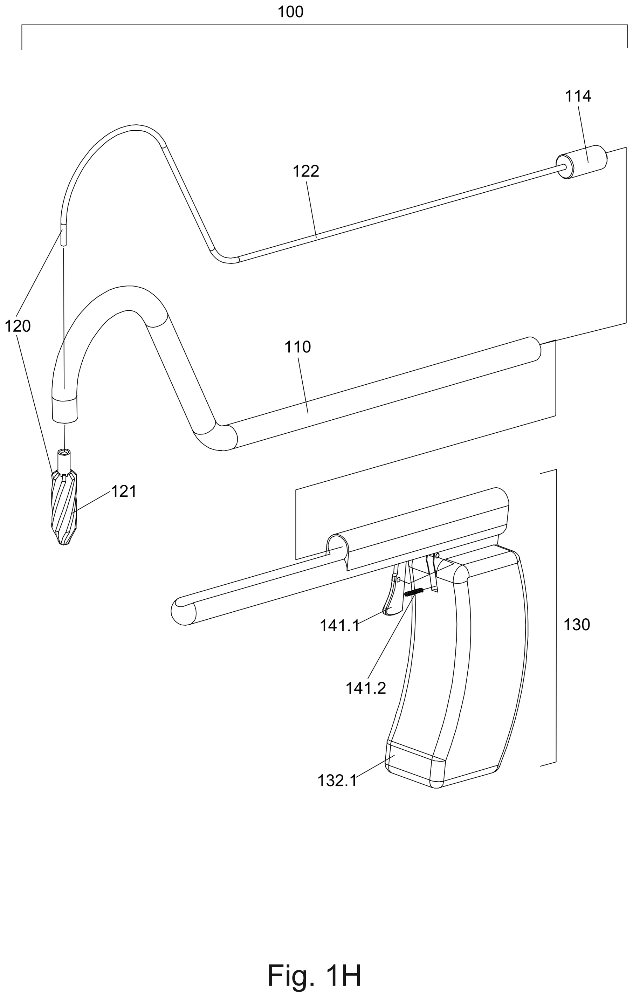

The table below provides the meaning of the different reference numbers included in Figures 1-11.

| Reference | Element |
|---|---|
| 1 | First segment of the bone tunnel |
| 2 | Second segment of the bone tunnel |
| 10 | Cortical fixing device |
| 20 | Suture bands |
| 30 | Augmentation tissue |
| 100 | Rupturing tool |
| 110 | Tubular main body (TMB) |
| 110.1 | Proximal portion of the TMB |
| 110.2 | Intermediate portion of the TMB |
| 110.3 | Distal portion of the TMB |
| 111 | Longitudinal groove or rail in the proximal portion (Embodiments A, B, and C) for housing the trigger |
| 111.1, 111.2 | Faces of the longitudinal groove |
| 112.1 | Third handle (Embodiment C) |
| 112.2 | Fourth handle (Embodiment D) |
| 113 | Longitudinal projection in the proximal portion of the TMB (Embodiment D) |
| 114 | Second attachment and/or coupling means |
| 115 | Rotation projection |
| 120 | Rupturing assembly |
| 121 | First rupturing element |
| 121.1 | First axis of rupture |
| 122 | Rupturing movement transfer element (MTE) |
| 122.1 | Proximal end of the MTE |
| 122.2 | Distal end of the MTE |
| 123 | First attachment and/or coupling means |
| 130 | Longitudinal pivot body |
| 130.1 | Pivot axis |
| 130.2 | First striking edge |
| 131.1 | Pivot element (Embodiment A) |
| 131.2 | Channel (Embodiment B) |
| 131.3 | Second rupturing element (Embodiment C) |
| 131.4 | Cannulated pivot element (Embodiment D) |
| 131.5 | Rupturing tip (Embodiment C) |
| 131.6 | Proximal end of the second rupturing element (Embodiment C) |
| 132.1 | First handle (Embodiment A) |
| 132.2 | Second handle (Embodiment B) |
| 133.1 | First through conduit (Embodiment A) |
| 133.2 | Second through conduit (Embodiment B) |
| 133.3 | Third through conduit (Embodiment C) |
| 133.31 | Longitudinal groove of the third through conduit (Embodiment C) |
| 133.4 | Fourth through conduit (Embodiment C) |
| 133.5 | Fifth through conduit (Embodiment D) |
| 133.51 | Longitudinal groove of the fifth through conduit (Embodiment D) |
| 133.52 | Perpendicular (semicircular) groove in the fifth through conduit |
| 133.6 | Sixth through conduit (Embodiment D) |
| 134 | Second attachment and/or coupling means |
| 140 | Sliding coupling means for the longitudinal pivot body |
| 141.1 | Trigger |
| 141.2 | Push spring |
| 141.3 | Tab |
| 200 | Rupturing guide |
| 210 | First tubular guide |
| 211 | First longitudinal conduit (of the first tubular guide) |
| 211.1 | First longitudinal guide axis |
| 211.2 | Proximal end of the first tubular guide |
| 211.3 | Distal end of the first tubular guide |
| 212 | Second striking edge |
| 220 | Second tubular guide |
| 221 | Second longitudinal conduit (of the second tubular guide) |
| 221.1 | Second longitudinal guide axis |
| 221.2 | Proximal end of the second tubular guide |
| 221.3 | Distal end of the second tubular guide |
| 222 | Third striking edge |
| 230 | Guide arch |
| 231 | Proximal portion of the guide arch |
| 231.1 | First sliding coupling means |
| 231.2 | Trigger of the first sliding coupling means |
| 231.3 | Groove of the first sliding coupling means |
| 232 | Distal portion of the guide arch |
| 232.1 | Second sliding coupling means |
| 232.2 | Trigger of the second sliding coupling means |
| 232.3 | Groove of the second sliding coupling means |
| 300 | First rupturing system |
| 400 | Second rupturing system |
| 1000 | Rupturing movement generator device (drill) |
| 1010 | Actuator of the rupturing movement generator device |
| 2000 | External bit |

### DETAILED DESCRIPTION OF THE INVENTION

Four preferred embodiments ("Embodiment A", "Embodiment B", "Embodiment C", and "Embodiment D") of the rupturing tool (100) according to the present invention will be described in detail below.

### First preferred embodiment of the rupturing tool of the invention

Figures 1A-1H show a first preferred embodiment of the rupturing tool (100) according to the present invention, referred to hereinafter as "Embodiment A". As seen in Figure 1A, this rupturing tool (100) comprises:
- a tubular main body (110) which in turn comprises: a longitudinal proximal portion (110.1), an intermediate portion (110.2), and a distal tip (110.3), wherein the intermediate portion (110.2) connects the longitudinal proximal portion (110.1) and the distal tip (110.3);
- a rupturing assembly (120) which in turn comprises:
   - a first rupturing element (121) which is configured to receive and perform a rupturing movement about a first axis of rupture (121.1); and
   - a movement transfer element (122), housed inside the main body (110), which is configured to receive and perform a rupturing movement and to transfer said rupturing movement to the first rupturing element (121); wherein the movement transfer element (122) comprises a proximal portion (122.1) and a distal portion (122.2); and wherein the movement transfer element (122) is attached to the first rupturing element (121) by the distal portion (122.2);
- a longitudinal pivot body (130), oriented substantially along a pivot axis (130.1); and
- sliding coupling means (140) adapted to allow the movement of the longitudinal pivot body (130) along the pivot axis (130.1).

Said rupturing tool (100) is characterized in that the pivot axis (130.1) and the axis of rupture (121.1) define a first angle (α) comprised substantially in the range between 20° and 170°, more preferably between 60° and 160°. Nevertheless, other embodiments of the rupturing tool (100) can have different values of the first angle (α).

As illustrated in Figure 1A, the axis of rupture (121.1) and the pivot axis (130.1) define a sagittal plane (101) of the rupturing tool (100) which contains both axes (121.1, 130.1) in any position of the rupturing tool (100). From the perspective of a user who grips the first handle (132.1), the sagittal plane (101) splits the rupturing tool (100) into two halves (to the "left" and to the "right", respectively, with respect to the pivot axis (130.1) according to the depiction thereof in this Figure 1A).

In more detail, as shown in Figure 1B, the longitudinal pivot body (130) further comprises:
- a longitudinal pivot element (131.1) arranged substantially along the pivot axis (130.1);
- a first handle (132.1) attached to the longitudinal pivot element (131.1); and
- a first through conduit (133.1) attached to the longitudinal pivot element (131.1); wherein said first through conduit (133.1) is adapted to house the longitudinal proximal portion (110.1) and to allow the linear movement of said longitudinal proximal portion (110.1) along the pivot axis (130.1).

In this embodiment, the pivot element (131.1) is substantially cylindrical.

In this embodiment, as also seen in Figure 1A, the male component of the sliding coupling means (140) is the longitudinal proximal portion (110.1) of the tubular main body (110), and the female component of said sliding coupling means (140) is the first through conduit (133.1).

The longitudinal pivot body (130) is configured to perform a linear movement, moving along the longitudinal proximal portion (110.1) of the tubular main body (110) from at least a first position to at least a second position, or vice versa.

In Figure 1B, the arrows correspond to the movement of certain elements when a user uses the tool. Therefore, the user can hold the first handle (132.1) and move the longitudinal pivot element (131.1) along the pivot axis (130.1) by means of actuating the trigger (141.1). Upon reaching the longitudinal position desired for said longitudinal pivot element (131.1), the user releases the trigger (141.1), which longitudinally blocks the rupturing tool (100).

In this embodiment, the proximal portion (110.1) of the tubular main body (110) is coupled to the rupturing movement generator device (1000) (not shown) by means of the second attachment and/or coupling means (114). Preferably, the second attachment and/or coupling means (114) are in the form of an elongated rod or cylinder.

As shown in Figures 1C and 1D, this rupturing tool (100) is adapted to swivel from a first position to at least a second position by means of the rotation of the sagittal plane (101) with respect to the pivot axis (130.1); such that after swiveling from the first position to the second position:
- the sagittal plane (101') in the at least second position defines a second angle (β) with respect to the sagittal plane (101) in the first position, and
- the first rupturing element (121) in the at least second position defines said second angle (β) with respect to the position of said first rupturing element (121) in the first position.

For example, sagittal plane (101) refers to the sagittal plane of the tool in standby, in a "neutral" position (without pivoting, as shown in Figure 1A); whereas sagittal plane (101') refers to the sagittal plane of the tool rotated according to the second angle, as can be seen in Figures 1C and 1D.

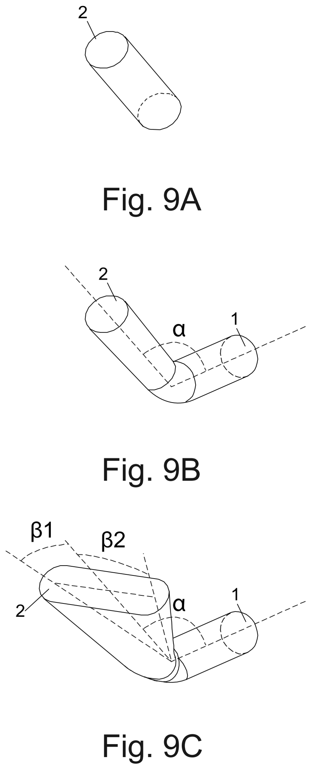

For example, Figure 1C shows how the tubular main body (110) can be pivoted to the "right" into a second position in which the sagittal plane (101') forms a certain angle β1 with respect to the sagittal plane (101) of the rupturing tool (100) in a neutral position observed in Figure 1A. Similarly, Figure 1D illustrates the tubular main body (110) being pivoted to the "left" a certain angle β2. In this way, the second angle (β) which defines the angular widening of a second segment (2) of the bone tunnel created with the tool will be equal to β1 + β2. This will be described in detail below in Figure 9.

Throughout the present document, "neutral position" shall be understood to mean a position in which β1=β2=0°.

Preferably, the second angle (β) (defined as β1+β2) is comprised between 70 and 120°, for example, based on the rotation previously established by a user of the tool. In some embodiments, β1=β2 (in absolute value). In alternative embodiments, β1 and β2 differ from one another.

As illustrated in detail in Figure 1E (perspective view) and Figure 1F (side view), the rupturing tool comprises sliding coupling means (140) for the longitudinal pivot body (130). The sliding coupling means (140) in turn comprise blocking means for the longitudinal pivot body (130) configured to act on said longitudinal pivot body (130) in one or more longitudinal positions along the pivot axis (130.1). In this way, said blocking means for the longitudinal pivot body (130) are configured to enable at least two positions of the longitudinal pivot body (130):
- a blocked position in which the longitudinal position of the longitudinal pivot body (130) is fixed with respect to the longitudinal proximal portion (110.1), and
- an unblocked position in which the longitudinal pivot body (130) can move linearly along the longitudinal proximal portion (110.1).

It should be pointed out that the physical position of the longitudinal pivot element (131.1) along the pivot axis (130.1) is geometrically designed to prevent impacting against the first rupturing element (121).

As can be seen in the detail of the cross-section to the left of Figure 1F, the tubular main body (110) comprises a longitudinal groove (111), also referred to hereinafter as rail. The longitudinal groove (111) comprises a plurality of faces (111.1, 111.2), particularly an upper face (111.1) and side faces (111.2). The longitudinal groove (111) interacts with a trigger (141.1), as will be described in detail below.

Figure 1G is a detailed view showing the operation of the blocking means for the longitudinal pivot body (130), which comprises:
- a trigger (141.1) adapted to fit into a longitudinal groove (111) of the longitudinal proximal portion (110.1); and
- a push spring (141.2) attached to the trigger (141.1), said push spring (141.2) being configured to exert an actuation force on the trigger (141.1) which keeps the longitudinal pivot body (130) in the blocked position pressing the tubular main body (110), when the trigger (141.1) is not pressed.

The trigger (141.1) is configured so that when a user presses it, it opposes the actuation force of the spring (142.1), allowing the unblocking of the pivot body (130) on the longitudinal groove (111), and so that said pivot body (130) can move longitudinally along the pivot axis (130.1).

The tubular main body (110) comprises the longitudinal groove (111) on a lower surface of said tubular main body (110). An upper tab (141.3) of the trigger (141.1) is housed in said longitudinal groove (111). When the user is not actuating the trigger (141.1), the longitudinal groove (111) and the trigger (141.1) are in contact with all the faces (111.1, 111.2) of said longitudinal groove (111), preventing the movement between both.

When the trigger (141.1) is actuated, the tab (141.3) no longer contacts the upper face (111.1) of the longitudinal groove (111), such that the tubular main body (110) can move in a sliding manner along the pivot axis (130.1); and at the same time the side faces of the tab (141.3) always contact the side faces (111.2) of the longitudinal groove (111).

In this way, the trigger (141.1) is always in contact with the side faces (111.2) in order to be guided in a single direction along the pivot axis (130.1). In contrast, the tab (141.3) only comes into contact with the upper face (111.1).

Therefore, when the tab (141.3) and the upper face (111.1) are in contact, the movement of the device along the pivot axis (130.1) is blocked. In contrast, when a user activates the trigger (141.1), the contact of the upper face (111.1) with the tab (141.3) is unblocked, which allows longitudinal movement along the longitudinal groove (11) and/or pivot axis (130.1).

In these preferred embodiments, the rupturing movement generator device (1000) is a drilling device or drill which is configured to generate and perform a rupturing movement, i.e., a rotary movement. Additionally, it is configured to transfer the rupturing movement to the movement transfer element (122).

Lastly, Figure 1H shows an exploded perspective view of the different elements of the rupturing tool (100) according to Embodiment A. Said figure shows how the movement transfer element (122) is attached to the first rupturing element (121) in a fixed manner. Said attachment is established by the distal portion (122.2) of the movement transfer element (122). Likewise, the movement transfer element (122) is coupled to the rupturing movement generator device (1000) by means of the second attachment and/or coupling means (114), which allows attaching the proximal portion (122.1) of the movement transfer element (122) to said drill and thereby transferring the rupturing movement.

### Second preferred embodiment of the rupturing tool (100) of the invention ("Embodiment B")

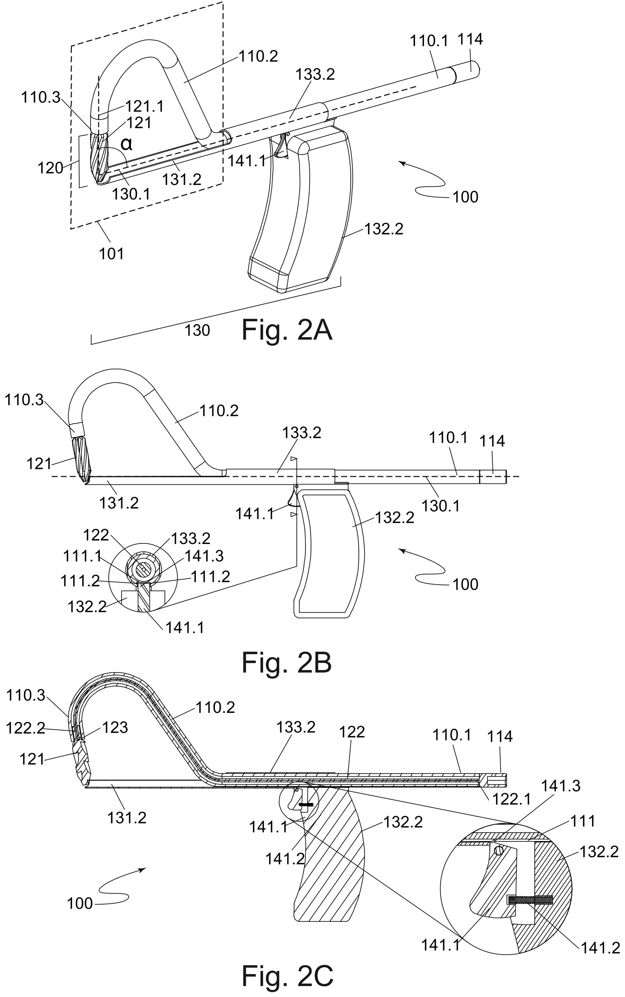

Figures 2A-D show a second preferred embodiment of the rupturing tool (100). Figure 2A corresponds to a perspective view, whereas Figures 2B-2C are side views.

In this second preferred embodiment of the invention, as can be seen in Figures 2A-2B, the longitudinal pivot body (130) further comprises:
- a longitudinal channel (131.2) which contains the pivot axis (130.1), wherein said longitudinal channel (131.2) comprises a concave face surrounding the longitudinal proximal portion (110.1) of the tubular main body (110); wherein the longitudinal channel (131.2) is adapted to slide along the longitudinal proximal portion (110.1);
- a second handle (132.2) attached to the longitudinal channel (131.2); and
- a second through conduit (133.2) attached to the longitudinal pivot element (131.1), wherein said second through conduit (133.2) is adapted to house the longitudinal proximal portion (110.1) and to allow the linear movement of said longitudinal proximal portion (110.1) along the pivot axis (130.1).

As can be seen in Embodiment B of Figures 2A and 2B, the male component of the sliding coupling means (140) is the proximal portion (110.1) of the tubular main body (110) and the female component of said sliding coupling means (140) is the second through conduit (133.2).

A user holds the second handle (132.2) and can pivot the main body about the pivot axis (130.1) at will between a plurality of positions for each of which the angulation of the sagittal plane (101, 101') of the rupturing tool (100) changes (i.e., the second angle (β) is modified).

The pivot axis (130.1) and the first rupturing element (121) are angled according to the first angle (α).

The longitudinal pivot body (130) is configured to perform a linear movement, moving along the longitudinal proximal portion (110.1) of the tubular main body (110) from at least a first position to at least a second position, or vice versa.

In this embodiment B, the blocking means for the longitudinal pivot body (130) comprise:
- a trigger (141.1) adapted to fit into a longitudinal groove (111) of the longitudinal proximal portion (110.1); and
- a push spring (141.2) attached to the trigger (141.1), said push spring (141.2) being configured to exert an actuation force on the trigger (141.1) which keeps the longitudinal pivot body (130) in the blocked position pressing the tubular main body (110), when the trigger (141.1) is not pressed.

As can be seen in the detail of the cross-section to the left of Figure 2B, the tubular main body (110) comprises a longitudinal groove (111) which in turn comprises a plurality of faces (111.1, 111.2), particularly an upper face (111.1) and side faces (111.2). The longitudinal groove (111) interacts with a trigger (141.1).

As can be seen in Figure 2C, the blocking of the tubular main body (110) along the pivot axis (130.1) is generated by the trigger (141.1) impacting against said tubular main body (110). This blocking system is similar to that shown above for Embodiment A. The trigger (141.1) of the second handle (132.2) is configured so that when a user presses it, it opposes the actuation force of the push spring (141.2), allowing the unblocking of the pivot body (130) on the longitudinal groove (111), and so that said pivot body (130) can move longitudinally along the pivot axis (130.1). In particular, the tubular main body (110) comprises the longitudinal groove (111) on a lower surface of said tubular main body (110). An upper tab (141.3) of the trigger (141.1) is housed in said longitudinal groove (111). When the user is not actuating the trigger (141.1), the longitudinal groove (111) and the trigger (141.1) are in contact with all the faces (111.1, 11.2) of said longitudinal groove (111), preventing the movement between both. When the trigger (141.1) is actuated, the tab (141.3) no longer contacts the upper face (111.1) of the longitudinal groove (111), such that the tubular main body (110) can move in a sliding manner along the pivot axis (130.1); and at the same time the side faces of the tab (141.3) always contact the side faces (111.2) of the longitudinal groove (111).

Like in Embodiment A, the trigger (141.1) is always in contact with the side faces (111.2) in order to be guided in a single direction along the pivot axis (130.1). In contrast, the tab (141.3) only comes into contact with the upper face (111.1).

Therefore, when the tab (141.3) and the upper face (111.1) are in contact, the movement of the device along the pivot axis (130.1) is blocked. In contrast, when a user activates the trigger (141.1), the contact of the upper face (111.1) with the tab (141.3) is unblocked, which allows longitudinal movement along the longitudinal groove (11) and/or pivot axis (130.1).

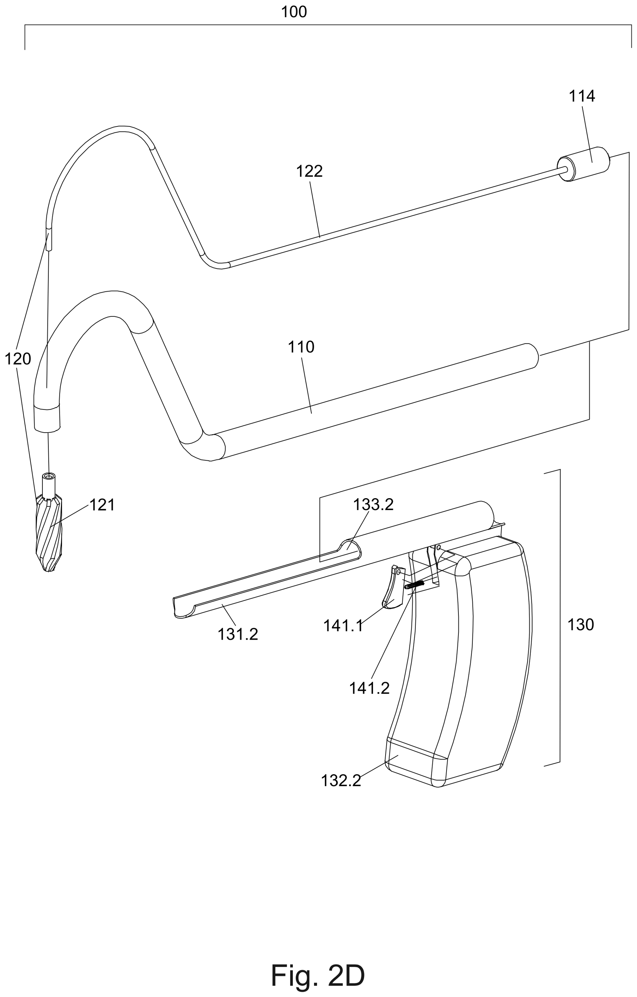

Lastly, Figure 2D is an exploded view of this embodiment of the rupturing tool (100).

### Third preferred embodiment of the rupturing tool of the invention ("Embodiment C")

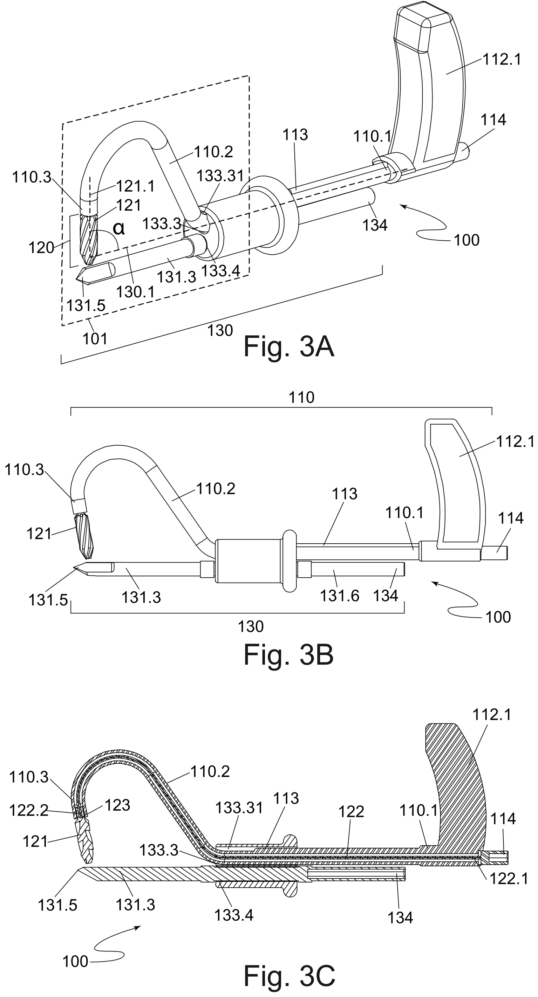

Figures 3A-3D show a third preferred embodiment of the rupturing tool (100). Figure 3A corresponds to a perspective view, whereas Figures 3B-3C are side views. As shown in these figures, the pivot body (130) further comprises:
- a third through conduit (133.3) which in turn comprises at least one longitudinal groove (133.31) adapted to fit at least one longitudinal projection (113) of the longitudinal proximal portion (110.1), the third through conduit (133.3) being adapted to house the longitudinal proximal portion (110.1) and to allow the linear movement of said longitudinal proximal portion (110.1) along the pivot axis (130.1);
- a fourth through conduit (133.4); and
- a second rupturing element (131.3), partially housed in the fourth through conduit (133.4) and adapted to project from said fourth through conduit (133.4), wherein said second rupturing element (131.3) comprises a second attachment and/or coupling means (134) which are adapted to attach or couple a proximal end (131.6) of the second rupturing element (131.3) to the rupturing movement generator device (1000) (not shown in Figures 3A-3D).

As can be seen in the figures, the tubular main body (110) further comprises a third handle (112.1) that allows a user to easily hold the rupturing tool (110) and to pivot the main body about the pivot axis (130.1) at will between a plurality of positions for each of which the angulation of the sagittal plane (101, 101') of the rupturing tool (100) changes (i.e., the second angle (β) is modified).

As can be seen in Figure 3B, the third through conduit (133.3) in turn comprises a longitudinal groove (133.31) where a longitudinal projection (113) of the longitudinal proximal portion (110.1) is housed. In this embodiment, the male component of the sliding coupling means (140) comprises the longitudinal proximal portion (110.1) and the longitudinal projection (113), whereas the female component of said sliding coupling means (140) comprises the third through conduit (133.3) and the longitudinal groove (133.31).

In this embodiment, the second rupturing element (131.3) is configured to rotate freely inside the fourth through conduit (133.4) about a fixed longitudinal position of said fourth through conduit (133.4). Additionally, the second rupturing element (131.3) is configured to move integrally with the pivot body (130) along the pivot axis (130.1).

Like in the first and second preferred embodiments of the invention, in this third preferred embodiment of the invention, the longitudinal pivot body (130) is configured to perform a linear movement, moving along the longitudinal proximal portion (110.1) of the tubular main body (110) from at least a first position to at least a second position, or vice versa.

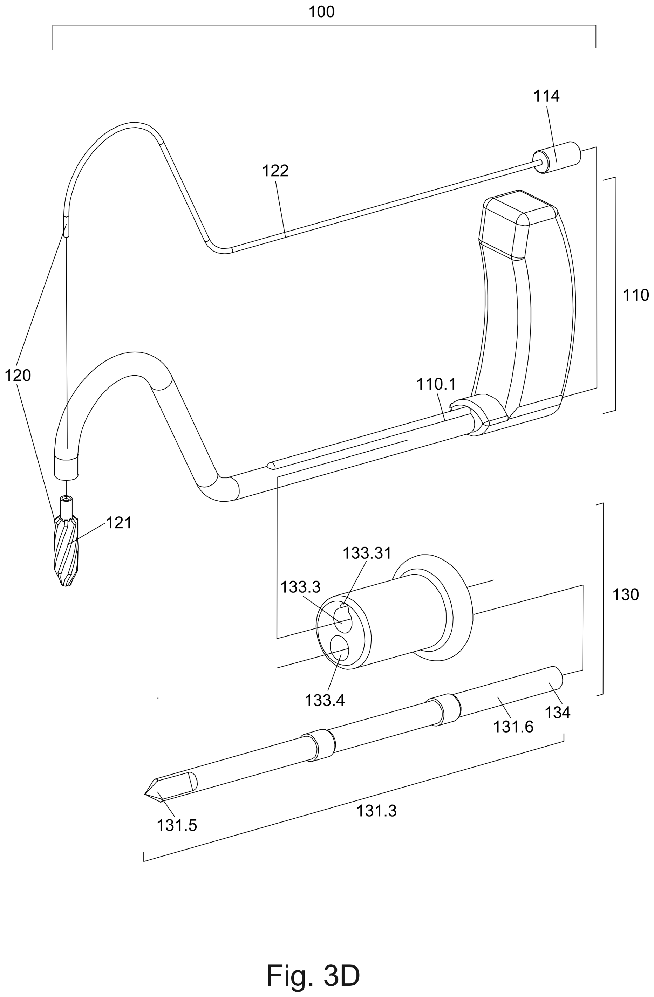

Figure 3D is an exploded view of this embodiment of the rupturing tool (100). Figure 3D shows, in greater detail, the longitudinal pivot body (130), which comprises a second rupturing element (131.3), in this case a built-in bit, and a second attachment and/or coupling means (134). The latter comprises the third through conduit (133.3) and the fourth through conduit (133.4), substantially parallel to one another.

Lastly, as can be seen in Figures 3A-3D, a rupturing tip (131.5) of the second rupturing element (131.2) comprises a configuration with a sharp distal tip or portion sized to be driven into the bone cortex. Nevertheless, other embodiments can have more than one sharp tip depending on the specific application for which the rupturing tool (100) will be used.

Although not shown in Figures 3A-3D, another variant of Embodiment C comprises blocking means for the longitudinal pivot body (140), similar to those described in detail for Embodiments A and B (with a trigger, etc.).

### Fourth preferred embodiment of the rupturing tool of the invention ("Embodiment D")

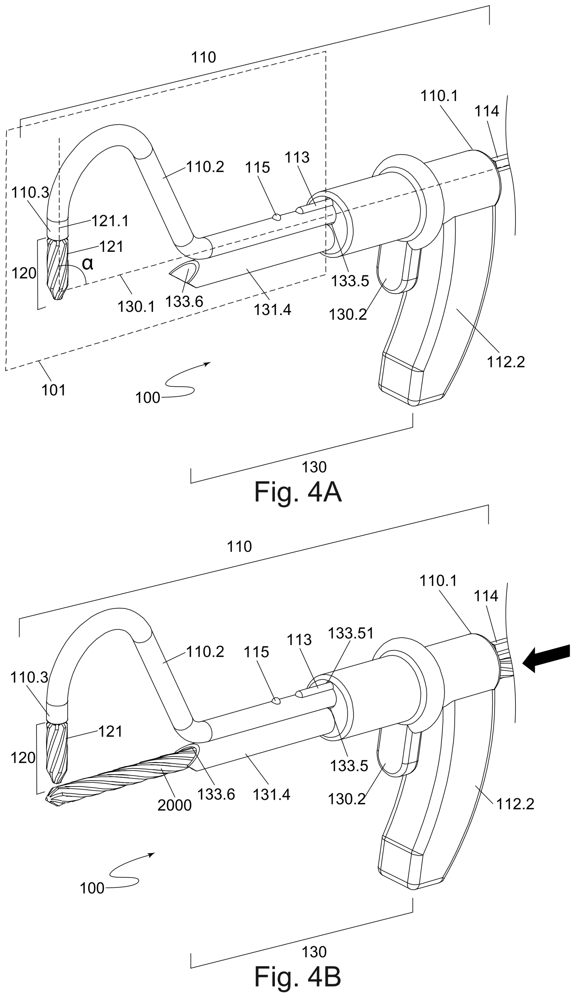

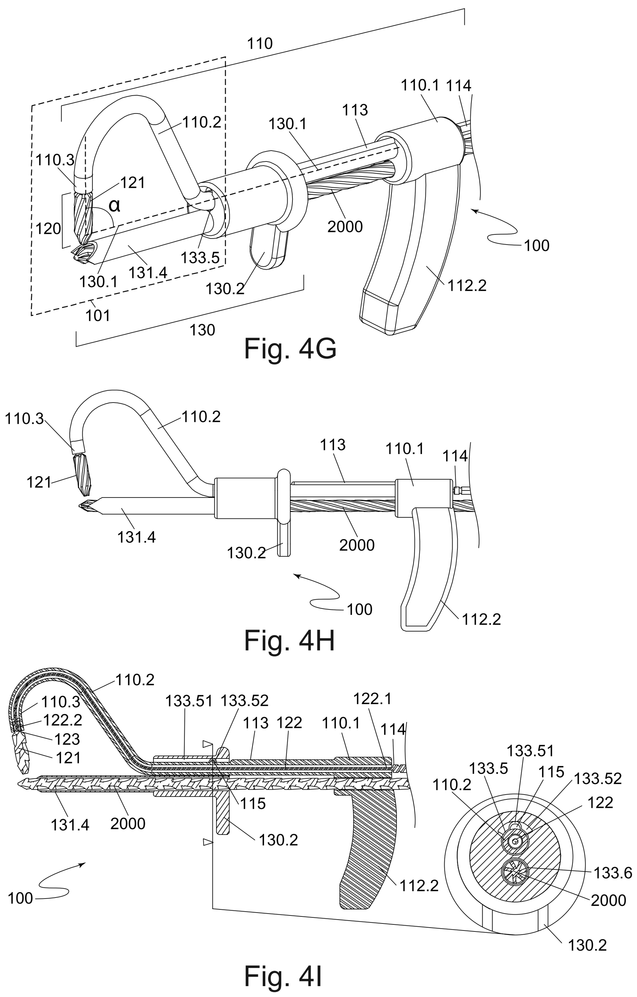

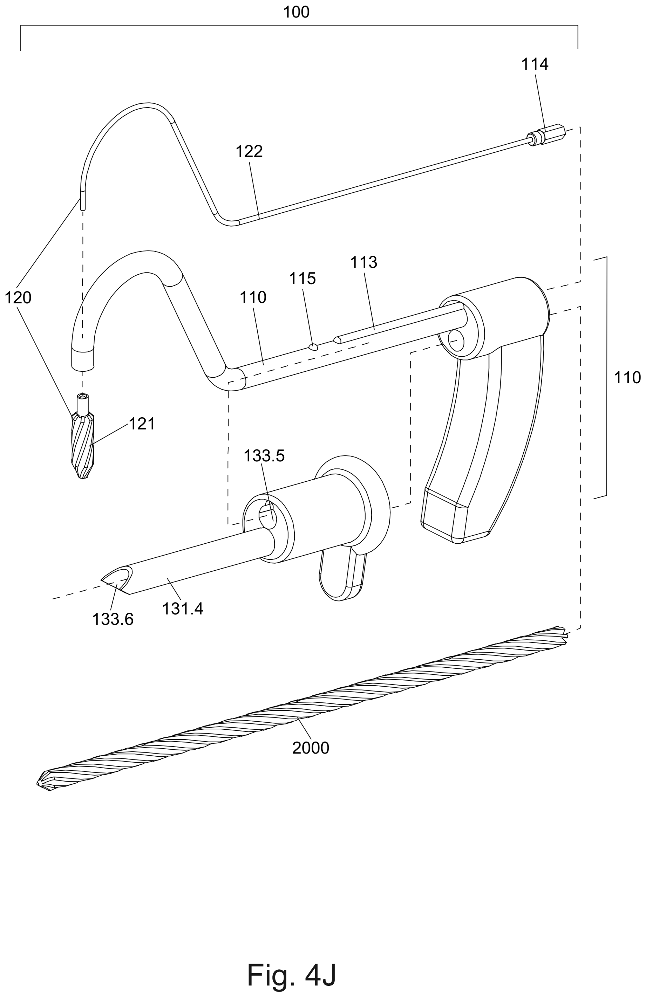

Figures 4A-4J show a fourth preferred embodiment of the rupturing tool (100). Figures 4A-4G correspond to a perspective view, whereas Figures 4H-4I are side views of said rupturing tool (100). Figure 4J is an exploded view of the rupturing tool (100) according to Embodiment D.

In this embodiment and as seen in Figure 4A, the longitudinal pivot (130) further comprises:
- a first striking edge (130.2), a prolongation of the longitudinal pivot body (130); wherein said first striking edge (130.2) is configured to receive a striking force in the direction of the pivot axis (130.1);
- a fifth through conduit (133.5) which in turn comprises at least one longitudinal groove (133.51) adapted to house the at least one longitudinal projection (113) of the longitudinal proximal portion (110.1); said fifth through conduit (133.5) also likewise comprises a perpendicular groove (133.52) adapted to house a rotation projection (115);
- a sixth through conduit (133.6) adapted to house and guide a bit (2000);

wherein the male component of the sliding coupling means (140) comprises the longitudinal proximal portion (110.1) and the longitudinal projection (113), whereas the female component of said sliding coupling means (140) comprises the fifth through conduit (133.5) and the longitudinal groove (133.51);
wherein the tubular main body (110) further comprises a fourth handle (112.2) attached to the longitudinal proximal portion (110.1); and
wherein the longitudinal pivot body (130) is configured to perform a linear movement, moving along the longitudinal proximal portion (110.1) from at least one rotation-blocking position to at least one rotation position, or vice versa, wherein:
   - if the longitudinal pivot body (130) is located in the at least one rotation-blocking position, the longitudinal groove (133.51) houses at least one portion of a longitudinal projection (113), and the perpendicular groove (133.52) does not house any portion of the longitudinal projection (113); and
   - if the longitudinal pivot body (130) is located in the at least one rotation position, the longitudinal groove (133.51) does not house any portion of the longitudinal projection (113), and the perpendicular groove (133.52) houses the rotation projection (115).

Therefore, in the rotation position, the longitudinal groove (133.51) has completely overcome the longitudinal projection (113); whereas in said rotation position, the perpendicular groove (133.52) houses the hemispherical rotation projection (115) of the longitudinal proximal portion (110.1), said rotation projection (115) being adapted to fit the longitudinal proximal portion (110.1) into the perpendicular groove (133.52) of the fifth through conduit (133.5).

In this way, when the rotation projection (115) (preferably hemispherical) is housed in the perpendicular groove (133.52), the tubular main body (110) can rotate a preestablished value of second angle (β) with respect to the pivot axis (130.1) in a manner guided and limited by the interaction between the perpendicular groove (133.52) and the rotation projection (115).

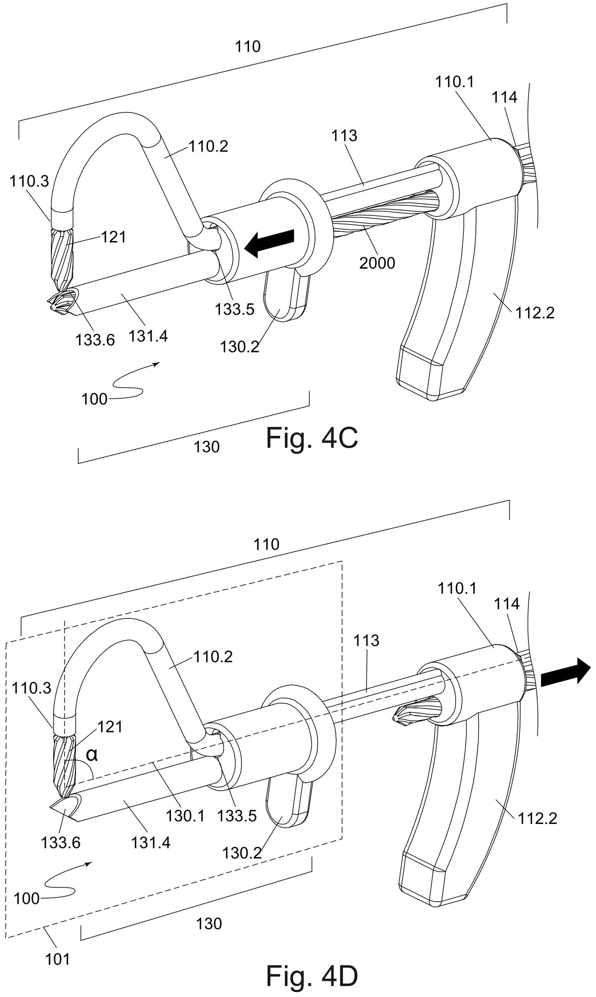

As shown in Figure 4B, a bit (2000) that goes through the proximal portion (110.1) and the sixth through conduit (133.6) and ends up protruding from the longitudinal pivot element (131.4) can be introduced in the rupturing tool (100). Furthermore, Figure 4B shows a position of the rupturing tool (100) in which the longitudinal projection (113) is housed in the longitudinal groove (133.51), such that the first striking edge (130.2) can move along the pivot axis (130.1), as indicated by means of arrows in Figures 4B and 4C. It should be pointed out that in this position the longitudinal groove (133.51) is separated from the rotation projection (115), such that the longitudinal pivot body (130) cannot rotate.

The function of the perpendicular groove (133.52) is to guide the pivoting of the sagittal plane (101) about the pivot axis (130.1), while at the same time limiting the second angle (β) of said pivoting. In this embodiment, in the rotation position, the perpendicular groove (133.52) (see the detail in Figure 4I) houses a hemispherical rotation projection (115) of the longitudinal proximal portion (110.1) which is adapted to fit into this perpendicular groove (133.52) of the fifth through conduit (133.5) of the longitudinal pivot body (130). Preferably, this perpendicular groove (133.52) is a complementary semicircular groove in the fifth through conduit (133.5) and extends perpendicular to the longitudinal groove (111) of the longitudinal pivot body (110). In this way, just as the longitudinal groove (111) allows moving the rupturing tool (100) forward and backward without rotation, this semicircular perpendicular groove (133.52) allows rotating this embodiment of the rupturing tool (100) without moving forward along the pivot axis (130.1).

It should be noted that, although Embodiment D comprises a preferably semicircular perpendicular groove (133.52), this technical function can be similarly performed by grooves having other curved, for example, elliptical, geometric shapes. Other alternative embodiments of Embodiment D comprise perpendicular grooves (133.52) having other geometric shapes. Therefore, in this context, "semicircular groove" or "perpendicular groove" should be understood in a broad manner, encompassing other rounded geometric shapes.

In a particular embodiment of this Embodiment D, a distal portion of the sixth through conduit (133.6) comprises a configuration with one or more sharp tips sized to be driven into the bone cortex.

To drive said tips into the bone cortex, the user must exert a certain striking force on a proximal end of the first striking edge (130.2) in the direction of the pivot axis (130.1). This force can be applied, for example, by means of a hammer.

Preferably, the proximal end of the first striking edge (130.2) is substantially flat.

Advantageously, the force applied to the first striking edge (130.2) ensures that the at least one tip is driven into the outer cortex of the bone. This allows the rupturing tool (100) to remain in a stable position, which increases the precision and usability of the tool when carving the bone tunnel.

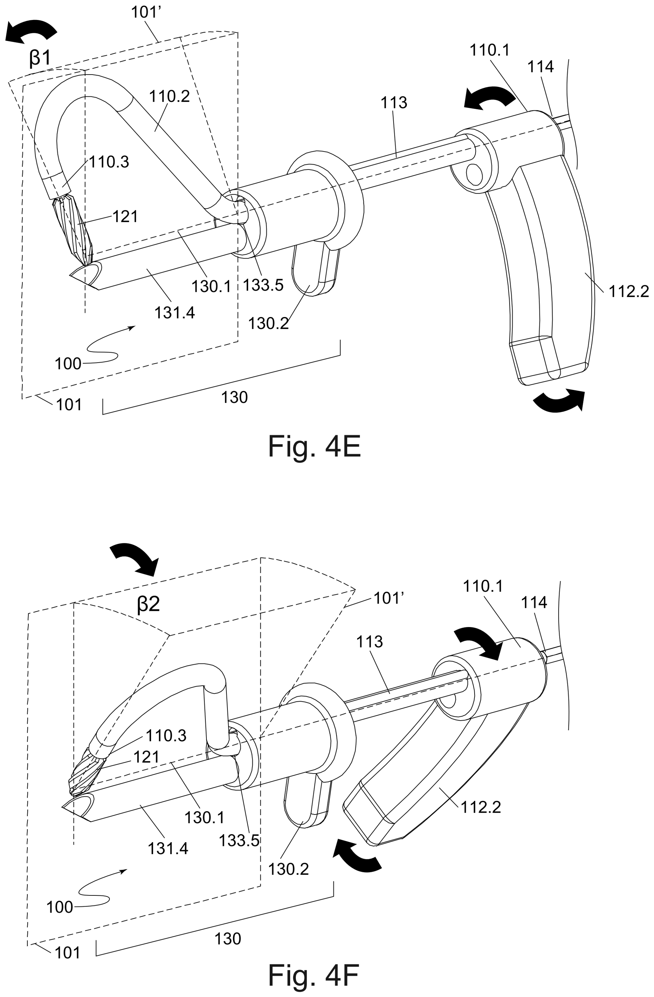

As can be seen in Figures 4E and 4F, and as shown in the figures of Embodiment A, the user can hold the fourth handle (112.2) and pivot the tubular main body (110) to the "right" into a second position in which the sagittal plane (101') forms a certain angle β1 with respect to the sagittal plane (101) of the rupturing tool (100) in a neutral position (shown in Figure 4D), see Figure 4E. The fourth handle (112.2) performs a function similar to that of the first, second, and third handles in Embodiments A, B, and C. As can be seen, reference (101') refers to the same sagittal plane (101), once the second angle rotates by the value. Similarly, Figure 4F illustrates the pivoting of the tubular main body (110) a certain angle β2 to the "left". In this way, the second angle (β) which defines the angular widening of a second segment (2) of the bone tunnel created with the tool will be equal to β1+β2, as will be analyzed below. In these figures, the arrows denote the direction of the movement.

It should be pointed out that these Figures 4E and 4F show the longitudinal pivot body (130) in a position in which the longitudinal groove (133.51) slidingly fits the at least one rotation projection (115) such that the longitudinal pivot body (130) can now rotate freely about the pivot axis (130.1) as indicated by means of the arrows, unlike what happened in Figure 4B.

Figures 4G-4H show how a bit (2000) is housed in said rupturing tool (100). In particular, the bit (2000) is introduced into the proximal portion (110.1) and the sixth through conduit (133.6), going through both and protruding from the longitudinal pivot element (131.4). The bit is directly coupled to a movement generator device (1000), this device not being shown in the figure.

Figure 4I illustrates the structure of the striking edge (133.2) in detail, including the fifth through conduit (133.5), the sixth through conduit (133.6), and the two grooves (133.51, 133.52) of the fifth through conduit (133.5). The bit (2000) is introduced into the sixth through conduit (133.6).

Lastly, Figure 4J corresponds to an exploded view of Embodiment D.

Though not shown in Figures 4A-4J, another variant of Embodiment D comprises blocking means for the longitudinal pivot body (140), similar to those described in detail for Embodiments A and B (with a trigger, etc.).

### Rupturing guide (200)

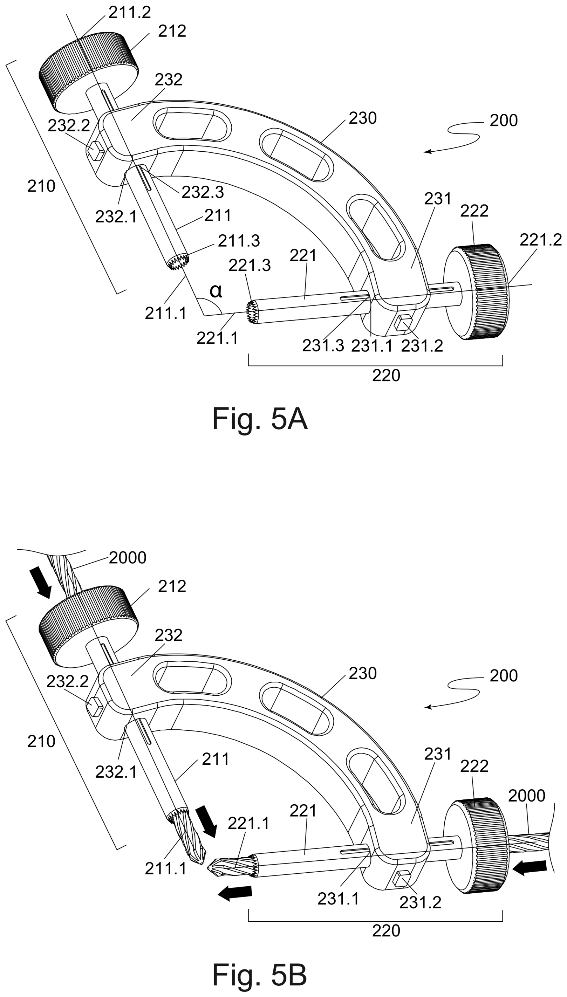

A rupturing guide (200) comprised in some embodiments of rupturing systems (300, 400) of the invention, as will be seen below, will be described next. The rupturing guide (200) is suitable for carrying out the prior milling of a bone. As illustrated in Figure 5A, said rupturing guide (200) comprises:
- a first tubular guide (210) which in turn comprises:
   ∘ a first longitudinal conduit (211) oriented according to a first longitudinal guide axis (211.1), wherein the first longitudinal conduit (211) comprises a proximal end (211.2) and a distal end (211.3); and
   ∘ a second striking edge (212), a prolongation of the first longitudinal conduit (211) at the proximal end (211.2); wherein the second striking edge (212) is configured to receive a striking force in the direction of the first longitudinal guide axis (211.1);
- a second tubular guide (220) which in turn comprises:
   ∘ a second longitudinal conduit (221) oriented according to a second longitudinal guide axis (221.1), wherein the second longitudinal conduit (221) comprises a proximal end (221.2) and a distal end (221.3); and
   ∘ a third striking edge (222), a prolongation of the second longitudinal conduit (221) at the proximal end (221.2); wherein the third striking edge (222) is configured to receive a striking force in the direction of the second longitudinal guide axis (221.1); and
- a guide arch (230) which comprises:
   ∘ a proximal arch portion (231) which in turn comprises a first sliding coupling means (231.1) adapted to couple and uncouple, in a fixed manner, the proximal arch portion (231) with respect to the first tubular guide (210) in a plurality of different positions along the first longitudinal guide axis (211.1); and
   ∘ a distal arch portion (232) which in turn comprises a second sliding coupling means (232.1) adapted to couple and uncouple, in a fixed manner, the distal arch portion (232) with respect to the second tubular guide (220) in a plurality of different positions along the second longitudinal guide axis (221.1);
wherein in the rupturing guide (200):
- the first longitudinal conduit (211) and the second longitudinal conduit (221) are adapted to accommodate a bit (2000), said bit (2000) being configured to move along said longitudinal conduits (211, 221);
- the first longitudinal guide axis (211.1) and the second longitudinal guide axis (221.1) form an angle substantially equal to the first angle (α) of the rupturing tool with respect to one another (100).

The first sliding coupling means (231.1) are adapted to couple the first tubular guide (210) to the proximal portion of guide arch (231). Similarly, the second sliding coupling means (232.1) are adapted to couple the second tubular guide (220) to the distal arch portion (232). In this embodiment, the sliding coupling means (231.1, 232.1) comprise respective longitudinal grooves (231.3, 232.3) and respective triggers (231.2, 232.2).

As a result of the rupturing guide (200), an initial bone tunnel (1, 2) wherein a first segment (1) and a second segment (2) of said tunnel are substantially straight and form an angle substantially equal to α can be created. Subsequently, and by means of the operation of the first rupturing element (121) of the rupturing tool (100), the second segment (2) of the tunnel is angularly widened.

The arrows in Figure 5B illustrate the movement of certain elements when the rupturing guide (200) is in use. In particular, drills (2000) can be introduced into the tubular guides (210, 220). The drills (2000) are coupled to the rupturing movement generator device (1000) such that, when this device is activated, rupturing movement is transferred from the rupturing movement generator device (1000) to said drills (2000), carving the bone and generating the two initially straight segments (1 ,2) of the bone tunnel, which form a first angle (α).

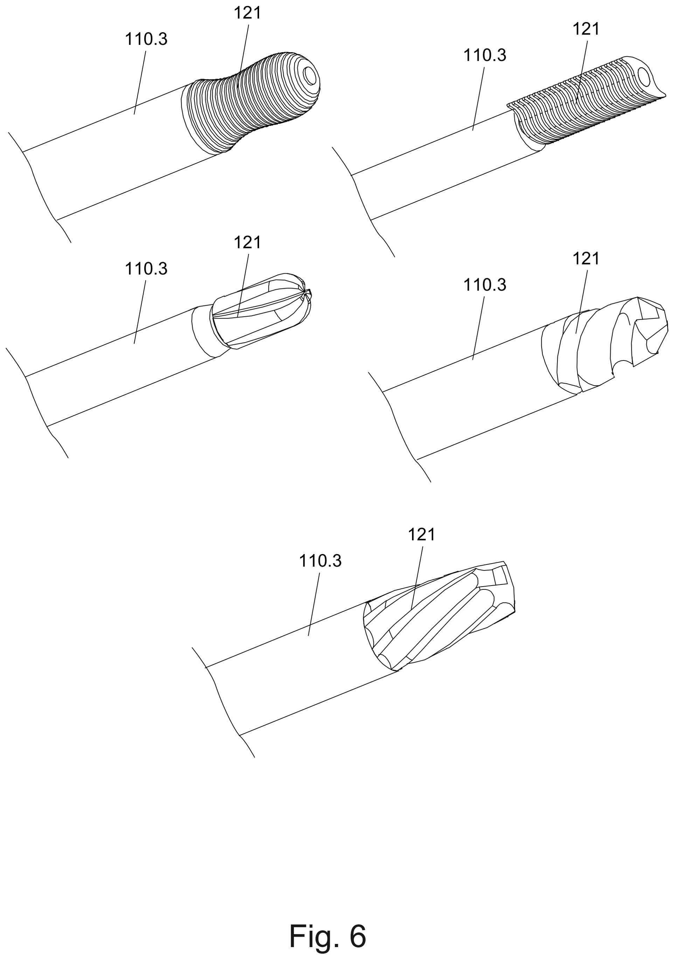

Figure 6 illustrates different geometries of the first rupturing element (121) of the rupturing tool (100) according to certain embodiments of the invention. As shown, these geometries comprise beveled recesses, polyhedral faces, a trilobular tip or appendage, etc. In rupturing tools (100) comprising more than one rupturing element, said additional rupturing elements can also present the geometries shown in this Figure 6.

### First rupturing system (300)

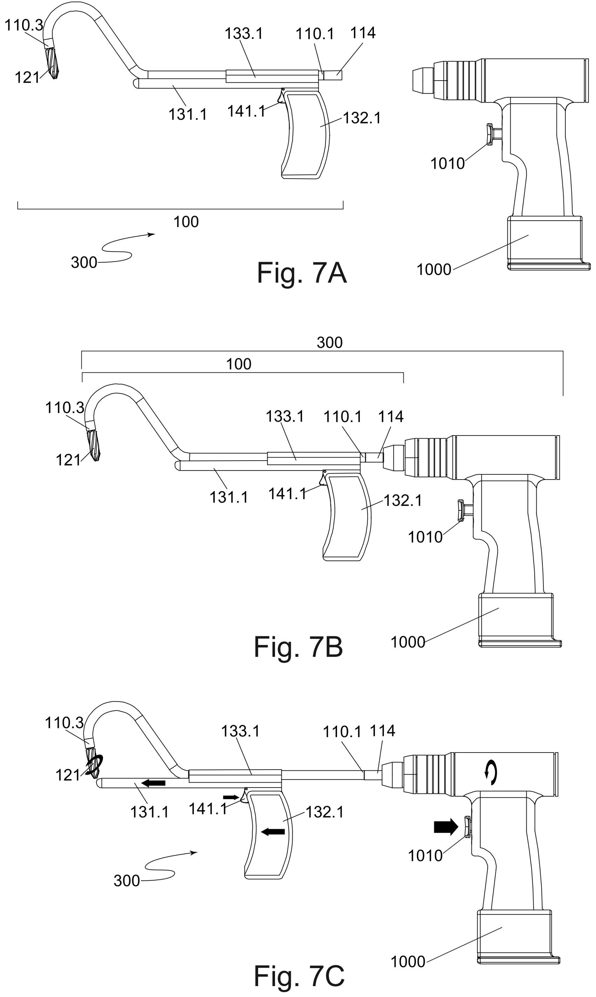

Figures 7A-7C correspond to a first rupturing system (300) according to the present invention. In particular, Figure 7A shows a side view of the system (300) with the rupturing tool (100) uncoupled from the rupturing movement generator device (1000); whereas Figure 7B shows a side view of the system (300) with the rupturing tool (100) coupled to the rupturing movement generator device (1000) which is inactive.

As can be seen in Figures 7A and 7B, the first rupturing system (300) comprises:
- a rupturing movement generator device (1000) configured to generate and perform a rupturing movement; and
- a rupturing tool (100) adapted to cooperate with the rupturing movement generator device (1000); said rupturing tool (100) being able to be coupled to the rupturing movement generator device (1000) by means of the movement transfer element (122). In this case, the movement transfer element (122) transmits a rotational movement generated by the rupturing movement generator device (1000) to the first rupturing element (121).

In Figures 7A and 7B, the movement transfer element (122) is inside the tubular main body (110), so it is not shown in said figures.

The rupturing movement generator device (1000), which is a drill in this case, comprises an actuator (1010) which serves to allow a user to activate or deactivate the rupturing movement at will. The rupturing movement generator device (1000) is further configured to transfer the rupturing movement to the movement transfer element (122). In this embodiment, the proximal portion (110.1) of the tubular main body (110) is coupled to the rupturing movement generator device (1000) by means of the second attachment and/or coupling means (114). Preferably, the second attachment and/or coupling means (114) are in the form of an elongated rod or cylinder.

In the embodiment of Figure 7, the rupturing movement generator device (1000) has been coupled to the rupturing tool (100) according to "Embodiment A" described in detail above. In this way, the rupturing tool (100) can be uncoupled from the drill as can be seen in Figure 7A or said rupturing tool can be coupled to the drill as shown in Figure 7B. Nevertheless, any other embodiment of the rupturing tool (100) is suitable for being coupled or uncoupled in a similar manner, granting greater versatility to the rupturing system (300).

Once the rupture generator device (1000) is coupled by means of the second attachment and/or coupling means (114), the position of the through conduit (133.1) along the pivot axis (130.1) can be adjusted by means of pressing the trigger (141.1) of the first handle (132.1).

Figure 7C illustrates by means of arrows the movement described by the elements of the rupturing system (300) when it is in use. Once the actuator (1010) is activated, the first rupturing element (121) is activated and starts to rotate, as shown by the arrows. Likewise, the user can rotate the tubular main body (110) and can also move the first through conduit (133.1) longitudinally along said pivot axis (131.1) while holding the first handle (132.1) and pressing the trigger (141.1).

In a particular embodiment, the rupturing system (300) comprises a disposable rupturing tool (100).

In another particular embodiment, the rupturing system (300) comprises a disposable rupturing tool (100) and a disposable rupturing movement generator device (1000), with the exception of the motor and the battery of the rupturing movement generator device (1000).

### Second rupturing system (400)

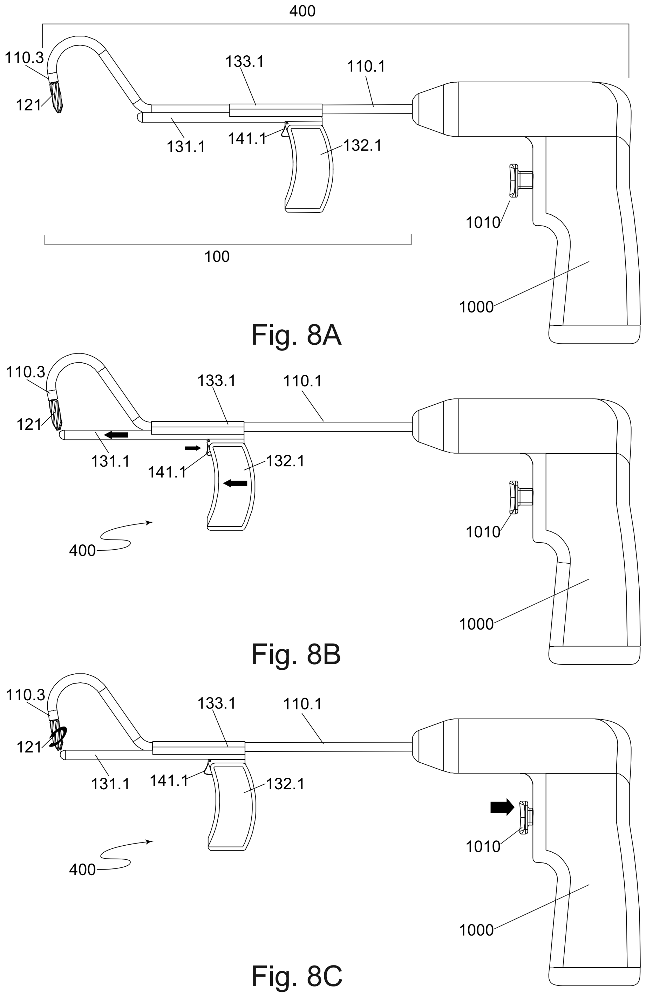

Figure 8A corresponds to a side view of a second rupturing system (400) according to the present invention, which comprises:
- a rupturing movement generator device (1000), for example, a drill; said drill comprising an actuator (1010) which allows the user to activate and deactivate it; and
- a rupturing tool (100) integrally attached to the rupturing movement generator device (1000) by means of the movement transfer element (122) and by means of the longitudinal proximal portion (110.1) of the tubular main body (110).

As can be seen in Figure 8A, the tubular main body (110) is directly integrated in the rupture generator device (1000). The movement transfer element (122) is inside both the rupture generator device (1000) and the tubular main body (110), so it is not shown in said figure.

Therefore, the rupturing movement generator device (1000) is configured to transfer the rupturing movement (in this case, a rotational movement) to the movement transfer element (122), which in turn transmits same to the first rupturing element (121).

As illustrated in Figure 8B, when the user presses the trigger (141.1) of the first handle (132.1), the first through conduit (133.1) can be moved along the pivot axis (130.1). Once the desired position is adjusted longitudinally, the actuator (1010) is activated as shown in Figure 8C, in which the arrows show the movement of the elements of the rupturing tool (100) in use. Therefore, the way in which the rupturing movement generator device (1000) generates the rupturing movement which is transmitted to the first rupturing element (121) through the longitudinal proximal portion (110.1) is observed.

In a preferred embodiment of any of the rupturing tools (100) or rupturing systems (300, 400) described above, the rupturing movement generated with the rupturing movement generator device (1000) is a rotary movement. More preferably, the rupturing movement is an oscillating rotary or vibratory movement.

In a particular embodiment, the first rupturing element (121) has a length of between 10 mm and 25 mm.

As mentioned above, a possible use of the rupturing tool (100) of the invention is to carve angled bone tunnels. Figures 9A-9C show in detail different segments (1, 2) of an angled bone tunnel carved in the greater tubercle of the humerus by means of the invention.
- Figure 9A: an initially straight second segment (2) of the bone tunnel is observed.
- Figure 9B: the second segment (2) of the bone tunnel connected to a straight first segment (1), which initially forms an angle of α degrees with respect to the second segment (2), is shown. Said angle coincides with the first angle (α) between the axis of rupture (121.1) and the pivot axis (130.1), according to the configuration of the rupturing tool (100).
- Figure 9C: the complete bone tunnel (1, 2) is illustrated with the intraarticular outlet opening widened by means of using any of the rupturing systems (300, 400) of the invention, whereby the initially straight second segment (2) is angularly widened. As can be seen, the second segment (2) is widened by a second angle (β).

As will be seen below, both segments (1, 2) of the tunnel are initially created with the first rupturing element (121) and then the second segment (2) is angularly widened by an angle β.

### Surgical method performed by means of the rupturing tool (100) and the rupturing systems (300, 400) of the present invention

In a fourth inventive aspect which is complementary to the first inventive aspect, the invention provides a surgical method for widening a second segment (2) (intraarticular outlet opening) of an angled bone tunnel (1, 2) carved in a human or animal body during the repair of a connective tissue, preferably in minimally invasive surgeries. The surgical method and three examples of use thereof which can be performed as a result of the invention will be described below.

Figure 10A describes, by way of a flow chart, the main steps of the surgical method according to the fourth inventive aspect, which allow carving an angled bone tunnel that in turn comprises a first segment (1), a second segment (2), and an angle α between both segments (1) and (2) of the angled bone tunnel (1, 2), said angle being substantially equal to the first angle (α) of the rupturing tool (100) and the intraarticular outlet opening of said second segment (2) of the bone tunnel can then be widened with a geometry similar to the geometry of the original anatomical area of insertion of a tendon being repaired. Therefore, according to Figure 10A, the following steps must be performed:
A) Providing a rupturing system (300, 400) such as the system of the present invention, wherein the rupturing tool (100) is coupled to a rupturing movement generator device (1000).
B) Carving an angled bone tunnel (1, 2), wherein both segments (1, 2) are angled with respect to one another by an angle substantially equal to the first angle (α) of the rupturing tool (100).
C) Placing the first rupturing element (121) in the second segment (2) of the angled bone tunnel (1,2).
D) Placing the distal portion of the longitudinal pivot body (130) in the first segment (1) of the angled bone tunnel (1,2).
E) Activating, by means of an actuator (1010), the rupturing movement generator device (1000) coupled to the rupturing tool (100); then, with the center at a point of the pivot axis (130.1), pivoting the rupturing tool to both sides by a second angle (β) (a second angle β1 and β2 in a first direction and in a direction opposite the first direction, for example, to the right and to the left about the pivot axis (130.1), respectively). The first rupturing element (121) therefore performs a windshield wiper-like movement which carves the bone and anatomically widens the second segment (2) of the bone tunnel.
F) Optionally, causing the rupturing tool (100) to move backward and/or forward to continue the anatomical widening of the second segment (2) of the bone tunnel.
G) Deactivating the rupturing system (300) by means of the actuator (1010); removing the distal portion of the longitudinal pivot body (130) from the first segment (1) of the bone tunnel (1,2), and removing the rupturing element from the second segment (2) of the bone tunnel (1,2).
H) Removing the rupturing tool (100) through the arthroscopic portal.

In certain embodiments of the surgical method according to Figure 10A, step F is omitted.

Other embodiments of the surgical method according to Figure 10 A do comprise the optional step F). In such embodiments, in this optional step, the first rupturing element (121) is caused to move backward in the distal-proximal direction and/ or the rupturing element (121) is caused to move forward in the proximal-distal direction, a predetermined distance while the rupturing system (300, 400) is activated, pivoting the rupturing tool (100) to the right and to the left about the pivot axis (130.1), i.e., to both sides of said pivot axis (130.1); thereby widening the outlet opening of the second segment (2) of the bone tunnel in one or more planes parallel to a first carving plane in which the opening was initially created in step E).

Step B) constitutes the main difference between the three examples of use which are described below. In particular, three options are described by way of example:
- Using the rupturing guide (200) in step B) for the creation of the angled bone tunnel (1, 2) and using the rupturing tool (100) according to Embodiments A and B starting from step C).
- Using the rupturing tool of Embodiment C in step B) for the creation of the first segment (1) of the angled bone tunnel and using this same rupturing tool starting from step C).
- Using the rupturing tool of Embodiment D in step B) for the creation of the first segment (1) of the angled bone tunnel and using this same rupturing tool starting from step C).

In the following examples of use, rupturing movement generator device (1000) should be understood to mean any device capable of carving a bone tunnel in a bone, for example, a surgical drill.

The flow chart of Figure 10A encompasses the first example of use (which, for example, can be performed by means of Embodiments A or B of the rupturing tool (100) of the invention); the second example of use (which, for example, can be performed by means of Embodiment C), and the third example of use (which, for example, can be performed by means of Embodiment D).

Throughout the present document, movement or rotation to the left or to the right or upward or downward should be understood to mean a movement seen from the perspective of the user who is handling the rupturing tool.

In a first example of use of the surgical method described in the flow chart of Figure 10A, an angled bone tunnel (1, 2) can be carved and anatomically widened in a bone by using a rupturing system (300, 400) comprising Embodiments A or B of the rupturing tool (100) and using the rupturing guide (200).

### Additional examples of possible uses of the surgical method of the invention

The first example of use of the method of the invention comprises performing the following steps:
A) Carving, by using the rupturing guide (200), an angled bone tunnel (1, 2) in a bone, wherein said tunnel in turn comprises: a first segment (1) of the bone tunnel and a second segment (2) of the bone tunnel; with an angle α between both segments being substantially equal to the first angle (α) of the rupturing tool (100). Initially, the segments (1, 2) are substantially straight.
B) Placing the rupturing element (121) of the rupturing tool (100) in the second segment (2) of the angled bone tunnel (1-2) created in the preceding step.
C) Moving the longitudinal pivot body (130) in the proximal-distal direction to a position in which at least one distal portion of the longitudinal pivot body is housed in the first segment of the angled bone tunnel (1-2).
D) Activating, by means of an actuator (1010), the rupturing movement generator device (1000) and then, by holding the first handle (Embodiment A) or the second handle (Embodiment B), rotating the tubular main body (110) in a first direction and in a second direction opposite the first direction about pivot axis (130.1), for example to the left and right on both sides of the pivot axis (130.1), such that the first rupturing element (121) carves the bone through a range of angular movement (second angle β); thereby creating a widened intraarticular outlet opening in the second segment (2), with a geometry similar to the geometry of the original anatomical area of insertion.
E) Stopping, by means of the actuator (1010), the rupturing movement generator device (1000) once the second segment (2) of the bone tunnel is anatomically widened in the desired manner; removing the longitudinal pivot body (130.1) from the first segment of the bone tunnel (1) and then removing the first rupturing element (121) from the widened second segment (2).

In a particular embodiment, the first example of use further comprises a step of causing the first rupturing element (121) to move backward in the distal-proximal direction and/or causing the first rupturing element (121) to move forward in the proximal-distal direction a predetermined distance, while the rupturing system (300, 400) is activated (when the rupturing movement generator device (1000) operates and rupturing movement is generated), simultaneously pivoting the rupturing tool (100) to the right and left about the pivot axis (130.1), thereby widening the outlet opening of the second segment (2) of the bone tunnel in one or more planes parallel to the carving plane which was initially created in step F).

In a second example of use of the surgical method described in the flow chart of Figure 10A, an angled bone tunnel (1, 2) can be carved and anatomically widened in a bone by using a rupturing system (300, 400) comprising Embodiment C of the rupturing tool (100).

The second example of use of the surgical method of the invention comprises performing the following steps:
A) Carving the initially straight second segment (2) of a bone tunnel (1, 2) angled towards the inside of the bone; then, with the longitudinal pivot body (130) being in the retracted position, placing the rupturing element (121) of Embodiment C of the rupturing tool (100) inside this second segment (2).
B) Coupling the rupturing movement generator device (1000) to the second rupturing element (131.3) and moving the longitudinal pivot body (130) in the proximal-distal direction such that the second rupturing element (131.3) carves the first segment (1) of the bone tunnel. The first segment (1) forms an angle (α) with respect to the second segment (2) of the bone tunnel, said angle being substantially equal to the first angle (α) of the rupturing tool (100).
C) Uncoupling the rupturing movement generator device (1000) from the second rupturing element (131.3), and then coupling same to the movement transfer element (122).
D) Activating, by means of an actuator (1010), the rupturing movement generator device (1000). Then, by holding the fourth handle (112.2), pivoting the longitudinal pivot body (130) to both sides of said pivot axis (130.1), i.e., to the left and to the right about the pivot axis (130.1). As a result of the pivoting, the first rupturing element (121) carves the bone through the range of angular movement in a carving plane and gradually widens the second segment (2) of the bone tunnel angularly, thereby creating a widened intraarticular outlet opening, with a geometry similar to the geometry of the original anatomical area of insertion of the tendon being repaired.
E) Stopping, by means of the actuator (1010), the rupturing movement generator device (1000) once the second segment (2) of the bone tunnel is carved with the desired angulation; removing the second rupturing element from the first segment (1) and then removing the first rupturing element (121) from the widened second segment (2).

Like in the first example of use, in a particular embodiment, the second example of use further comprises a step of causing the first rupturing element (121) to move backward in the distal-proximal direction and/ or causing said first rupturing element (121) to move forward in the proximal-distal direction a predetermined distance, while the rupturing system (300, 400) is activated, simultaneously pivoting the rupturing tool to the right and left about the pivot axis (130.1), thereby widening the outlet opening of the second bone tunnel (2) in one or more planes parallel to at least one carving plane which was created in step D).

Advantageously, in this second example of use, the rupturing tool (100) itself comprises a second rupturing element (131.3) which allows carving an angled bone tunnel from a first segment (1) of the bone tunnel.

In a particular embodiment, step A) of creating the second segment of the bone tunnel can be performed with any tool capable of punching and/or carving a bone tunnel with these characteristics.

In a third example of use of the surgical method described in the flow chart of Figure 10A, an angled bone tunnel (1, 2) can be carved and anatomically widened in a bone by using a rupturing system (300, 400) comprising Embodiment D of the rupturing tool (100).

The third example of use of the surgical method of the invention comprises performing the following steps:
A) Carving the initially straight second segment (2) of a bone tunnel (1, 2) angled towards the inside of the bone; then, with the longitudinal pivot body (130) being in the retracted position, placing the rupturing element (121) of Embodiment D of the rupturing tool (100) inside this second segment (2).
B) Coupling a bit (2000) to a rupturing movement generator device (1000) and passing same through the cannulated pivot element (131.4) to carve a first segment (1) of the bone tunnel which forms an angle (α) with respect to the second segment (2) of the bone tunnel.
C) Extending the cannulated pivot element (131.4) of the longitudinal pivot body (130) to a position in which it is housed in the first segment (1) of the bone tunnel.
D) Uncoupling the bit (2000) from the rupturing movement generator device (1000) and coupling the device to the movement transfer element of the rupturing tool (100).
E) Activating the rupturing movement generator device (1000), and while holding the fourth handle (112.2), causing the longitudinal pivot body (130) to pivot to both sides of said pivot axis (130.1). Therefore, the first rupturing element (121) carves the bone and gradually widens the second segment (2) of the bone tunnel angularly.
F) Stopping the rupturing movement generator device (1000) once the second segment (2) of the bone tunnel is carved with the desired angulation; removing the distal portion of the longitudinal pivot body (130) from the first segment (1) and then removing the first rupturing element (121) from the widened second segment (2).

Like in the two preceding examples of use, in a particular embodiment, the third example of use further comprises a step of causing the first rupturing element (121) to move backward in the distal-proximal direction and/ or causing said first rupturing element (121) to move forward in the proximal-distal direction a predetermined distance, while the rupturing system (300, 400) is activated, simultaneously pivoting the rupturing tool to the right and left about the pivot axis (130.1), thereby widening the outlet opening of the second bone tunnel (2) in one or more planes parallel to at least one carving plane which was created in step E).

In a particular embodiment, step A) of creating the second segment of the bone tunnel can be performed with any tool capable of die-cutting and/or carving a bone tunnel with these characteristics.

Figure 10B shows in detail a musculoskeletal depiction of the bone tunnel (1, 2) carved with one of the systems (300, 400) of the invention and the insertion of a graft for repairing a supraspinatus muscle tendon of the shoulder joint. In particular, Figure 10B shows:
1) Side and front views of a straight first portion (1) of the bone tunnel.
2) Side and front views of the initially straight second portion (2) of the bone tunnel, in which the angle formed by the first and second portions of the bone tunnel can be seen.
3) Side, front, and perspective views of the complete bone tunnel (1, 2), in which the second portion (2) has been angularly widened.

In the particular example of repairing the damaged supraspinatus muscle tendon of the rotator cuff, the dimensions of the intraarticular outlet opening of the bone tunnel to be created must be such that they allow the insertion of the end of the damaged tendon which, at the height of the rotator chord, has measurements of between 4 mm and 5 mm in thickness and between 20 mm and 25 mm in width. However, these particular measurement ranges provided must be adapted based on each anatomy and on the final application for which the rupturing tool (100) is used which, in a general use, is suitable for rupturing any connective tissue and/or cartilage tissue and/or bone tissue, in medicine or veterinary science.

Figures 10C and 10D illustrate an example of use of the rupturing tool (100) according to preferred Embodiment A of the invention for repairing the supraspinatus tendon of the shoulder joint. The arrows illustrate the movement of certain elements of the rupturing tool (100) when they are in use. In this example, the rupturing tool (100) is used together with a rupturing guide (200). Sequentially, the carving of the bone tunnel (1, 2) occurs as follows:
- First, as seen in Figure 10C, the rupturing guide (200) is used for carrying out a prior milling of the angled bone tunnel and generating the two initially straight segments (1, 2) of the tunnel which form an angle α. This rupturing guide has already been described above in relation to Figure 5. The use of a bit (2000), which is introduced into the tubular guides (210, 220), is also required. Briefly, the user places the rupturing guide (200) on the bone, as shown in Figure 10C (top panel). Then, as illustrated in Figure 10C (intermediate panel), a certain striking force is applied on the second striking edge (212) of the first longitudinal guide (210). In this way, and as a result of the bit (2000) which is housed inside the first tubular guide (210), the second segment (2) of the bone tunnel is carved. Next, as can be seen in Figure 10C (bottom panel), the operation is repeated by applying a striking force on the third striking edge (222) of the second longitudinal guide (220) to carve the first segment (1) of the tunnel.
- Then, as illustrated in Figure 10D (top panel), the first rupturing element (121) is inserted into the second segment (2) of the tunnel.
- Next, as seen in Figure 10D (intermediate panel), the longitudinal pivot element (131.1) is introduced into the first segment (1) of the bone tunnel. To move the pivot element (131.1), the user acts on the trigger (141.1) of the first handle (132.1) and moves it in the proximal-distal direction along the pivot axis (130.1).
- Subsequently, and as can be seen in Figure 10D (bottom panel), a movement generator device (1000) coupled to the rupturing tool (100) is activated, and while holding the first handle (132.1), the longitudinal pivot body (130) is caused to pivot to both sides of said pivot axis (130.1). Therefore, the first rupturing element (121) carves the bone and gradually widens the second segment (2) of the bone tunnel angularly.
- Lastly, as can be seen in Figure 10G, the rupturing tool (100) is removed from the created bone tunnel (1, 2) and the end of the supraspinatus muscle tendon being repaired is sutured and the suture bands (20) are passed through the carved bone tunnel (1, 2); and said suture bands (20) are retained by means of a cortical fixing device (10) which can be, for example, the one described in US patent 5,630,824 or the one described in European patent EP3141216 B1.

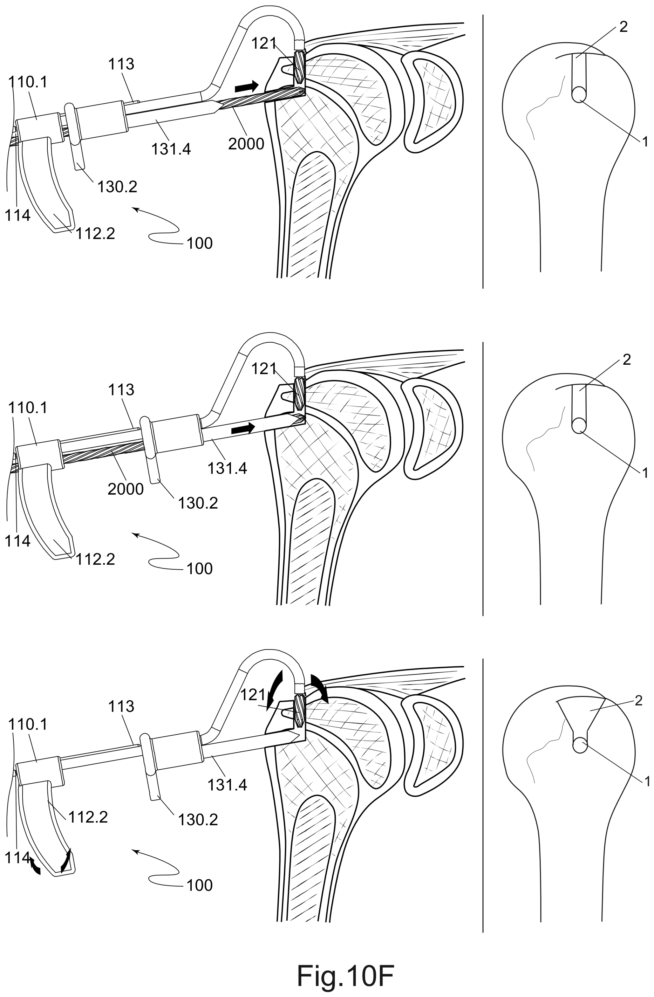

Figures 10E, 10F, and 10G show another example of use of the rupturing tool (100) according to preferred Embodiment D of the invention for repairing the supraspinatus tendon of the shoulder joint. Each of these figures shows frontal and side views of the bone tunnel that is being carved, together with the operation of the rupturing tool (100) during said carving. The arrows illustrate the movement of certain elements of the rupturing tool (100) when they are in use. Sequentially, the carving of the bone tunnel (1, 2) occurs as follows:
- First, as seen in Figure 10E (bottom panel), the bone is perforated to generate a second segment (2) of the bone tunnel. To that end, an external bit or another perforation element is used.
- Next, as shown in Figure 10F (top panel), and by using a bit (2000) that goes through the cannulated pivot element (131.4), a first segment (1) of the bone tunnel which forms an angle (α) with respect to the second segment (2) of the bone tunnel is carved. The bit (2000) is introduced through the proximal portion (110.1) of the tubular main body (110).
- Then, and as shown in Figure 10F (intermediate panel), the cannulated pivot element (131.4) of the longitudinal pivot body (130) is extended to a position in which it is housed in the first segment (1) of the bone tunnel. To extend the longitudinal pivot body (130), the user exerts a certain striking force on the first striking edge (130.2) in the direction of the pivot axis (130.1). Once the cannulated pivot element (131.4) is extended, it is fixed longitudinally as a result of the longitudinal projection (113) in the proximal portion of the tubular main body (110), which acts as a blocking mechanism and causes the cannulated pivot element (131.4) to be retained in the first segment (1) of the bone tunnel.
- Subsequently, and as can be seen in Figure 10F (bottom panel), a movement generator device (1000) coupled to the rupturing tool (100) is activated, and while holding the fourth handle (112.2), the longitudinal pivot body (130) is caused to pivot to both sides of said pivot axis (130.1). Therefore, the first rupturing element (121) carves the bone and gradually widens the second segment (2) of the bone tunnel angularly. During this operation, the cannulated pivot element (131.4) remains in the first segment (1) of the bone tunnel. The introduction of the cannulated pivot element (131.4) into the first segment (1) of the bone tunnel provides stability and precision to the rupturing tool during the anatomical widening of the second segment (2) of the bone tunnel.
- Lastly, as can be seen in Figure 10G, the rupturing tool (100) is removed and the end of the supraspinatus muscle tendon being repaired is sutured and the suture bands (20) are passed through the carved bone tunnel (1, 2); and said suture bands (20) are retained by means of a cortical fixing device (10) which can be, for example, the one described in US patent 5,630,824 or the one described in European patent EP3141216 B1.

The cannulated pivot element (131.4) is configured to be introduced into the body of the patient, said element being sharp in certain embodiments of the invention.

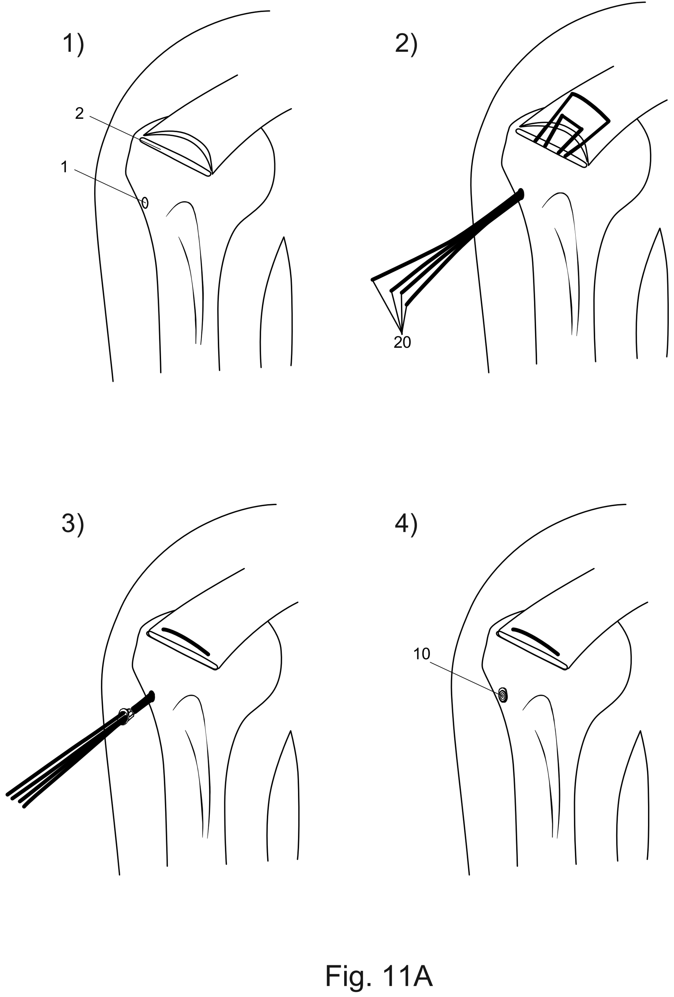

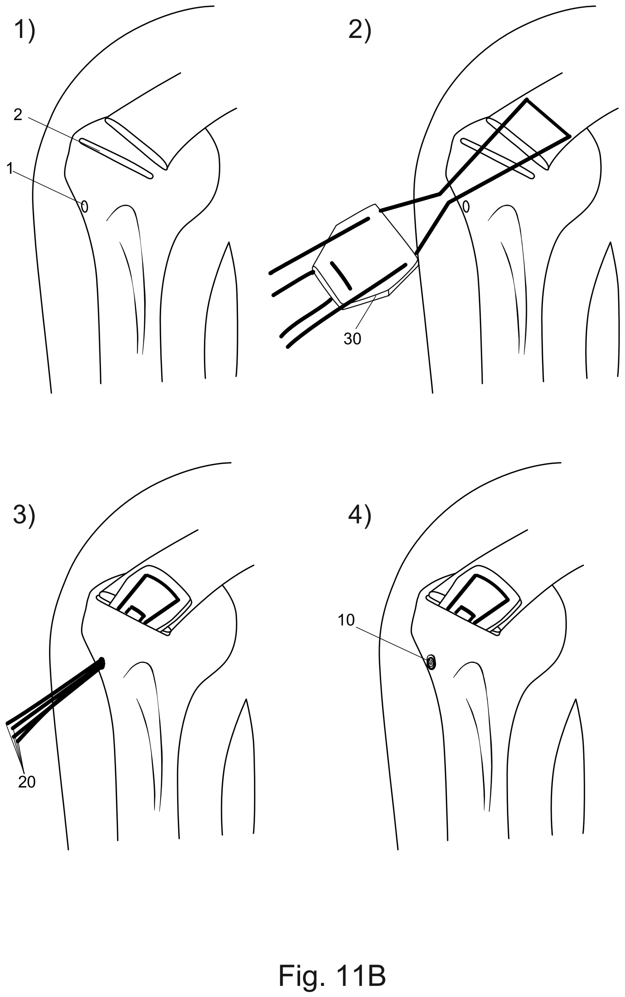

Figure 11A shows a general view of the steps of a first particular example of application of the surgical method of the present invention for repairing a partial rupture of the supraspinatus muscle tendon by means of an angled bone tunnel created and anatomically widened by means of any of the embodiments of the invention. This example requires the creation of an angled bone tunnel (1, 2) created by means of any of the rupturing systems (300, 400) of the invention, as can be seen in the top left panel of Figure 11A. Once the angled bone tunnel (1, 2) is created and the intraarticular outlet opening is widened into a fan or funnel by means of any of the rupturing systems (300, 400) of the invention, the end of the supraspinatus muscle tendon being repaired is sutured and the suture bands (20) are passed through the bone tunnel (1, 2), as seen in the top right panel ofFigure 11A. Subsequently, the end of the tendon is passed through the anatomically widened opening of the bone tunnel and the suture bands (20) are protruding from the bone tunnel (1, 2), as illustrated in the bottom left panel of Figure 11A. Lastly, the bottom right panel of Figure 11Ashows the suture bands (20) retained by means of a cortical polysuture fixing device (10) which can be, for example, the one described in US patent 5,630,824 or the one described in European patent EP3141216 B1.

Figure 11B shows a general view of the steps of a second particular example of application of the surgical method of the invention for repairing a complete rupture of the supraspinatus muscle tendon by means of an angled bone tunnel (1, 2) created and widened by means of any of the embodiments of the invention. This second example, like the first example, requires the creation of an angled bone tunnel (1, 2) created and anatomically widened by means of any of the rupturing systems (300, 400) of the invention, and the use of augmentation tissue (30) and suture bands (20) with which the end of the augmentation tissue (30) is passed through the anatomically widened opening of the bone tunnel. In a particular embodiment, the augmentation tissue (30) is a decellularized dermal tissue. In another particular embodiment, the augmentation tissue (30) comprises biodegradable biopolymer fibers. In another particular embodiment, the augmentation tissue (30) comprises a bioabsorbable poly(lactic-co-glycolic acid) (PLGA) aligned microfiber scaffold.

Lastly, like in the preceding example, the bottom right panel of Figure 11B shows the suture bands (20) retained by means of a cortical polysuture fixing device (10) which can be, for example, the one described in US patent 5,630,824 or the one described in European patent EP3141216 B1.

Taking into account that failure rates of up to 68% are reported for complete supraspinatus muscle tendon ruptures *(*Jost B, Pfirrmann CWA, Gerber C. Clinical outcome after structural failure of rotator cuff repairs. J Bone Joint Surg Am 2000; 82:304-14*.),* in all cases, the objective is to achieve revascularization of the end of the tendon through the osseointegration of the augmentation tissue in the created and anatomically widened angled bone tunnel.

## Claims

1. Rupturing tool (100) for minimally invasive surgical interventions, comprising:
- a tubular main body (110) which in turn comprises: a longitudinal proximal portion (110.1), an intermediate portion (110.2), and a distal tip (110.3), wherein the intermediate portion (110.2) connects the longitudinal proximal portion (110.1) and the distal tip (110.3);
- a rupturing assembly (120) which comprises:
∘ a first rupturing element (121) which is configured to receive and perform a rupturing movement about a first axis of rupture (121.1); and
∘ a movement transfer element (122), housed inside the main body (110), which is configured to receive and perform a rupturing movement and to transfer said rupturing movement to the first rupturing element (121); wherein the movement transfer element (122) comprises a proximal portion (122.1) and a distal portion (122.2); and wherein the movement transfer element (122) is attached to the first rupturing element (121) by the distal portion (122.2);
- a longitudinal pivot body (130), oriented substantially along a pivot axis (130.1); and
- sliding coupling means (140) adapted to allow the movement of the longitudinal pivot body (130) along the pivot axis (130.1); wherein said sliding coupling means (140) comprise at least a first component and a second component; the first component being adapted to be housed at least partially in the second component and to be able to move inside the second component, or vice versa, the second component being adapted to move while housing the first component;
wherein in said rupturing tool (100):
- the pivot axis (130.1) and the axis of rupture (121.1) define a first angle (α) comprised substantially in the range between 20° and 170°; and
- the axis of rupture (121.1) and the pivot axis (130.1) define a sagittal plane (101) of the rupturing tool (100) which contains both axes (121.1, 130.1) in any position of the rupturing tool (100); and
wherein the rupturing tool (100) is adapted to swivel from a first position to at least a second position by means of the rotation of the sagittal plane (101) with respect to the pivot axis (130.1); such that after swiveling from the first position to the second position:
- the sagittal plane (101) in the at least second position defines a second angle (β) with respect to the sagittal plane (101) in the first position, and
- the first rupturing element (121) in the at least second position defines said second angle (β) with respect to the position of said first rupturing element (121) in the first position.

2. Rupturing tool (100) according to the preceding claim, wherein the movement transfer element (122) further comprises at least a first attachment and/or coupling means (123) configured to attach and/or couple the movement transfer element (122) to the first rupturing element (121); and wherein said movement transfer element (122) is attached to a second attachment and/or coupling means (114) configured to attach and/or couple the movement transfer element (122) to a rupturing movement generator device (1000).

3. Rupturing tool (100) according to any of the preceding claims, wherein the sliding coupling means (140) for the longitudinal pivot body (130) further comprise blocking means for the longitudinal pivot body (130) configured to act on said longitudinal pivot body (130) in one or more longitudinal positions along the pivot axis (130.1), and wherein said blocking means are configured to enable at least two positions of the longitudinal pivot body (130):
- a blocked position in which the longitudinal position of the longitudinal pivot body (130) is fixed with respect to the longitudinal proximal portion (110.1), and
- an unblocked position in which the longitudinal pivot body (130) can move linearly along the longitudinal proximal portion (110.1).

4. Rupturing tool (100) according to any of claims 1-3, wherein the longitudinal pivot body (130) further comprises:
- a longitudinal pivot element (131.1) arranged substantially along the pivot axis (130.1);
- a first handle (132.1) attached to the longitudinal pivot element (131.1); and
- a first through conduit (133.1) attached to the longitudinal pivot element (131.1); wherein said first through conduit (133.1) is adapted to house the longitudinal proximal portion (110.1) and to allow the linear movement of said longitudinal proximal portion (110.1) along the pivot axis (130.1);
wherein the first component of the sliding coupling means (140) is a male component, and the second component of the sliding coupling means is a female component; and
wherein the male component of the sliding coupling means (140) comprises the longitudinal proximal portion (110.1) of the tubular main body (110), and the female component of said sliding coupling means (140) comprises the first through conduit (133.1).

5. Rupturing tool (100) according to any of claims 1-3, wherein the longitudinal pivot body (130) further comprises:
- a longitudinal channel (131.2) which contains the pivot axis (130.1), wherein said longitudinal channel (131.2) comprises a face surrounding the longitudinal proximal portion (110.1) of the tubular main body (110); and wherein the longitudinal channel (131.2) is adapted to slide along the longitudinal proximal portion (110.1);
- a second handle (132.2) attached to the longitudinal channel (131.2); and
- a second through conduit (133.2) attached to the longitudinal pivot element (131.1), wherein said second through conduit (133.2) is adapted to house the longitudinal proximal portion (110.1) and to allow the linear movement of said longitudinal proximal portion (110.1) along the pivot axis (130.1);
wherein the first component of the sliding coupling means (140) is a male component, and the second component of the sliding coupling means is a female component; and
wherein the male component of the sliding coupling means (140) comprises the longitudinal proximal portion (110.1) of the tubular main body (110), and the female component of said sliding coupling means (140) comprises the second through conduit (133.2).

6. Rupturing tool (100) according to any of claims 3 to 5, wherein the blocking means for the longitudinal pivot body (130) comprise:
- a trigger (141.1) adapted to fit into a longitudinal groove (111) of the longitudinal proximal portion (110.1); and
- a push spring (141.2) attached to the trigger (141.1), said push spring (141.2) being configured to exert an actuation force on the trigger (141.1) which keeps the longitudinal pivot body (130) in the blocked position pressing the tubular main body (110), when the trigger (141.1) is not pressed;
and wherein the trigger (141.1) is configured so that when a user presses it, it opposes the actuation force of the spring (142.1), allowing the unblocking of the pivot body (130) on the longitudinal groove (111), and so that said pivot body (130) can move longitudinally along the pivot axis (130.1).

7. Rupturing tool (100) according to any of claims 1-3, wherein the pivot body (130) further comprises:
- a third through conduit (133.3) which in turn comprises at least one longitudinal groove (133.31) adapted to fit at least one longitudinal projection (113) of the longitudinal proximal portion (110.1), the third through conduit (133.3) being adapted to house the longitudinal proximal portion (110.1) and to allow the linear movement of said longitudinal proximal portion (110.1) along the pivot axis (130.1);
- a fourth through conduit (133.4); and
- a second rupturing element (131.3), partially housed in the fourth through conduit (133.4) and adapted to project from said fourth through conduit (133.4), wherein said second rupturing element (131.3) comprises a second attachment and/or coupling means (134) which are adapted to attach or couple a proximal end (131.6) of the second rupturing element (131.3) to the rupturing movement generator device (1000); and
wherein:
- the tubular main body (110) further comprises a third handle (112.1);
- the second rupturing element (131.3) is configured to rotate freely inside the fourth through conduit (133.4) about a fixed longitudinal position of said fourth through conduit (133.4),
- the second rupturing element (131.3) is configured to move integrally with the pivot body (130) along the pivot axis (130.1),
- the first component of the sliding coupling means (140) is a male component, and the second component of the sliding coupling means is a female component; and
- the male component of the sliding coupling means (140) comprises the longitudinal proximal portion (110.1) and the longitudinal projection (113); and the female component of said sliding coupling means (140) comprises the third through conduit (133.3) and the longitudinal groove (133.31).

8. Rupturing tool (100) according to any of claims 1-3, wherein the longitudinal pivot body (130) further comprises:
- a first striking edge (130.2), a prolongation of the longitudinal pivot body (130); wherein said first striking edge (130.2) is configured to receive a striking force in the direction of the pivot axis (130.1);
- a fifth through conduit (133.5) which in turn comprises at least one longitudinal groove (133.51) adapted to fit at least one longitudinal projection (113) of the longitudinal proximal portion (110.1); and said fifth through conduit (133.5) also comprises a perpendicular groove (133.52) adapted to house a rotation projection (115);
- a sixth through conduit (133.6) adapted to house and guide a bit (2000);
wherein the first component of the sliding coupling means (140) is a male component, and the second component of the sliding coupling means is a female component; and
wherein the male component of the sliding coupling means (140) comprises the longitudinal proximal portion (110.1) and the longitudinal projection (113); and the female component of said sliding coupling means (140) comprises the fifth through conduit (133.5) and the longitudinal groove (133.51);
wherein the tubular main body (110) further comprises a fourth handle (112.2) attached to the longitudinal proximal portion (110.1); and
wherein the longitudinal pivot body (130) is configured to perform a linear movement, moving along the longitudinal proximal portion (110.1) from at least one rotation-blocking position to at least one rotation position, or vice versa, wherein:
- if the longitudinal pivot body (130) is located in the at least one rotation-blocking position, the longitudinal groove (133.51) houses at least one portion of a longitudinal projection (113), and the perpendicular groove (133.52) does not house any portion of the longitudinal projection (113); and
- if the longitudinal pivot body (130) is located in the at least one rotation position, the longitudinal groove (133.51) does not house any portion of the longitudinal projection (113), and the perpendicular groove (133.52) houses the rotation projection (115).

9. Rupturing tool (100) according to any of the preceding claims, wherein the first axis of rupture (121.1) and the pivot axis (130.1) are arranged in the rupturing tool (100):
- defining a fixed first angle (α), or
- defining a variable first angle (α) according to a predetermined pattern.

10. Rupturing tool (100) according to claims 1 and 2, which further comprises a second attachment and/or coupling means (114) configured to attach and/or couple, in a fixed position, the longitudinal proximal portion (110.1) of the tubular main body (110) to the rupturing movement generator device (1000); such that in such situation, the rupturing tool (100) is integrally attached to the rupturing movement generator device (1000).

11. Rupturing tool (100) according to any of the preceding claims, wherein at least the distal portion (122.2) of the movement transfer element (122) is a flexible longitudinal element housed in the intermediate portion (110.2).

12. - Rupturing tool (100) according to any of the preceding claims, wherein the first rupturing element (121) is straight.

13. - Rupturing tool (100) according to any of the preceding claims, wherein the rupturing movement is rotary, oscillating rotary and/or vibrating about the first rupturing axis (121.1).

14. Rupturing system (300) for minimally invasive surgical interventions, comprising:
- a rupturing movement generator device (1000) configured to generate and perform a rupturing movement; and
- at least one rupturing tool (100) according to any of the preceding claims adapted to cooperate with the rupturing movement generator device (1000); said rupturing tool (100) being able to be coupled to the rupturing movement generator device (1000) at least by a means of the movement transfer element (122);
and wherein the rupturing movement generator device (1000) is further configured to transfer the rupturing movement to the movement transfer element (122).

15. Rupturing system (400) for minimally invasive surgical interventions, comprising:
- a rupturing movement generator device (1000) configured to generate and perform a rupturing movement; and
- a rupturing tool (100) according to any of the preceding claims integrally attached to the rupturing movement generator device (1000) by means of the movement transfer element (122) and by means of the longitudinal proximal portion (110.1);
wherein the rupturing movement generator device (1000) is further configured to transfer the rupturing movement to the movement transfer element (122).

16. Rupturing system (300, 400) according to any of claims 14 or 15, wherein the rupturing movement generated and performed with the rupturing movement generator device (1000) is a rotational movement.

17. The rupturing system (300, 400) according to any of claims 14-16, which further comprises a rupturing guide (200) adapted to carve a bone tunnel, wherein the rupturing guide (200) comprises:
- a first tubular guide (210) which in turn comprises:
o a first longitudinal conduit (211) oriented according to a first longitudinal guide axis (211.1), wherein the first longitudinal conduit (211) comprises a proximal end (211.2) and a distal end (211.3); and
∘ a second striking edge (212), a prolongation of the first longitudinal conduit (211) at the proximal end (211.2); wherein the second striking edge (212) is configured to receive a striking force in the direction of the first longitudinal guide axis (211.1);
- a second tubular guide (220) which in turn comprises:
∘ a second longitudinal conduit (221) oriented according to a second longitudinal guide axis (221.1), wherein the second longitudinal conduit (221) comprises a proximal end (221.2) and a distal end (221.3); and
∘ a third striking edge (222), a prolongation of the second longitudinal conduit (221) at the proximal end (221.2); wherein the third striking edge (222) is configured to receive a striking force in the direction of the second longitudinal guide axis (221.1); and
- a guide arch (230) which comprises:
∘ a proximal arch portion (231) which in turn comprises a first sliding coupling means (231.1) adapted to couple and uncouple, in a fixed manner, the proximal arch portion (231) with respect to the first tubular guide (210) in a plurality of different positions along the first longitudinal guide axis (211.1); and
∘ a distal arch portion (232) which in turn comprises a second sliding coupling means (232.1) adapted to couple and uncouple, in a fixed manner, the distal arch portion (232) with respect to the second tubular guide (220) in a plurality of different positions along the second longitudinal guide axis (221.1);
wherein in the rupturing guide (200):
- the first longitudinal conduit (211) and the second longitudinal conduit (221) are adapted to accommodate a bit (2000), said bit (2000) being configured to move along said longitudinal conduits (211, 221);
- the first longitudinal guide axis (211.1) and the second longitudinal guide axis (221.1) form an angle substantially equal to the first angle (α) of the rupturing tool with respect to one another.
